# EUROPEAN PATENT APPLICATION

(11) **EP 4 703 360 A1**
(43) Date of publication of application: **04.03.2026**
(21) Application number: 24796200.4
(22) Date of filing: 26.04.2024
(51) Int. Cl.: C07D 473/18, C12N 15/13, A61K 48/00, A61K 31/713, A61P 25/00, A61P 35/00

(54) **INTERMEDIATE FOR PREPARING OLIGONUCLEOTIDE ANALOG OR SALT THEREOF, AND PREPARATION METHOD THEREFOR**

(30) Priority: 27.04.2023 CN 202310469801
(71) Applicant: Shanghai Senhui Medicine Co., Ltd., Shanghai 201203 (CN); Jiangsu Hengrui Pharmaceuticals Co., Ltd., Lianyungang, Jiangsu 222047 (CN)
(72) Inventor: ZOU, Yang, Shanghai 201203 (CN); ZHU, Lingjian, Shanghai 201203 (CN); GAO, Shusan, Shanghai 201203 (CN); TAO, Weifeng, Shanghai 201203 (CN); HUANG, Jian, Shanghai 201203 (CN)
(74) Representative: Dragotti & Associati S.P.A.
(86) International application number: PCT/CN2024/089929
(87) International publication number: WO 2024/222826

(57) **Abstract**

An intermediate for preparing an oligonucleotide analogue or a salt thereof, and a preparation method therefor. In particular, the present invention relates to a compound of formula VIII and a preparation method therefor. The definitions of substituents are as defined in the description.

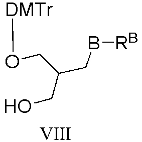

## Description

### Technical Field

The disclosure belongs to the field of medicine and relates to an intermediate required for preparing an oligonucleotide analogue or a salt thereof, and a preparation method therefor.

### Background Art

Oligonucleotides and analogues thereof are widely used in modern molecular biotechnology and biopharmaceutical research. They have been developed for various uses in molecular biology, including as a probe, a primer, a linker, an adapter and a gene fragment, and have important application value in biomedical science research. Commercial drug production requires a large amount (in kilograms) of oligonucleotides. Conventionally, the chemical synthesis of oligonucleotides is costly, and the initial product is low in purity and difficult to purify, which is very unfavourable for clinical research and large-scale drug production.

For example, patent WO 2022028462 discloses a method for preparing an oligonucleotide analogue represented by formula 1-6a. The method is costly and cumbersome to operate, involves difficult chiral resolution, and has a low product yield.

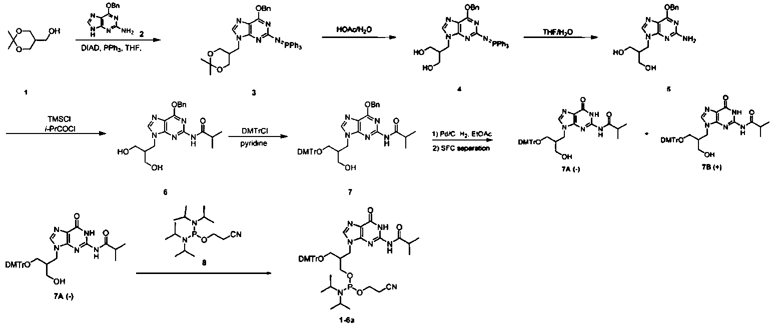

Therefore, developing a simple, economical and high-yield large-scale industrial production method for an oligonucleotide is a technical problem that needs to be solved urgently.

### Summary of the Invention

In order to overcome the deficiencies of the prior art, the objective of the disclosure is to provide an intermediate for preparing an oligonucleotide analogue, and a preparation method therefor.

The disclosure provides a preparation method for preparing an oligonucleotide analogue such as a compound of formula VIII or a salt thereof, 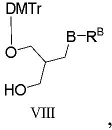 wherein the method comprises the step of converting a compound of formula II into a compound of formula VII, 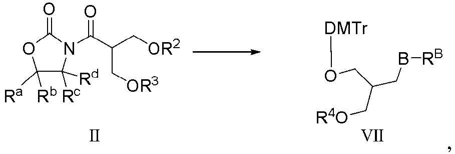 wherein
R^{a}, R^{b} and R^{c} are the same or different, and are each independently selected from hydrogen, halogen, hydroxyl, amino, C₁₋₆ alkyl, C₁₋₆ alkoxy, 3- to 10-membered cycloalkyl, 3- to 10-membered heterocyclyl, 6- to 10-membered aryl or 5- to 10-membered heteroaryl, wherein the alkyl, alkoxy, cycloalkyl, heterocyclyl, aryl and heteroaryl are optionally substituted with one or more substituents selected from halogen, hydroxyl, amino, C₁₋₆ alkyl or C₁₋₆ alkoxy;
R^{d} is selected from benzyl, phenyl, biphenyl or C₁₋₆ alkyl, wherein the benzyl, phenyl, biphenyl and C₁₋₆ alkyl are optionally substituted with one or more substituents selected from halogen, hydroxyl, amino, C₁₋₆ alkyl or C₁₋₆ alkoxy;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl;
R² and R⁴ are the same or different, and are each independently a hydroxyl protecting group;
R³ is selected from trityl, tert-butyldimethylsilyl, tert-butyldiphenylsilyl, (trimethylsilyl)ethyl or 2-tetrahydropyran.

Further, in some embodiments, R² is selected from benzyl, trityl, tert-butyldimethylsilyl, tert-butyldiphenylsilyl, (trimethylsilyl)ethyl or 2-tetrahydropyran, preferably benzyl.

Further, in some embodiments, R³ is selected from trimethylsilyl or trityl.

Further, in some embodiments, R⁴ is selected from trityl, tert-butyldimethylsilyl, tert-butyldiphenylsilyl, (trimethylsilyl)ethyl or 2-tetrahydropyran, preferably tert-butyldimethylsilyl or tert-butyldiphenylsilyl.

Further, in some embodiments, B is a substituted or unsubstituted base or base analogue, wherein the base or base analogue is selected from a purine base, a pyrimidine base, indole, 5-nitroindole or 3-nitropyrrole;

B is selected from substituted or unsubstituted purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole, preferably substituted or unsubstituted purine, adenine, guanine, 2,6-diaminopurine, 6-dimethylaminopurine, 2-aminopurine, cytosine, uracil, thymine, indole, 5-nitroindole and 3-nitropyrrole, and most preferably substituted or unsubstituted purine, adenine, guanine, 2,6-diaminopurine, 6-dimethylaminopurine and 2-aminopurine.

Further, in some embodiments, B is substituted or unsubstituted guanine, which is optionally substituted with a substituent selected from the group consisting of: halogen, hydroxyl, amino, nitro, cyano, carboxyl, oxo, -C(O)-C₁₋₆ alkyl, C₁₋₆ alkyl, -C(O)-C₁₋₆ alkoxy and C₁₋₆ alkoxy.

Further, in some embodiments, R^{B} is C(O)-C₁₋₆ alkyl, preferably -C(O)-CH₃, - C(O)-CH₂CH₃ or -C(O)-CH(CH₃)₂, and most preferably -C(O)-CH(CH₃)₂. In some embodiments, the method for preparing the compound of formula VIII or the salt thereof comprises the step of converting the compound of formula II into a compound of formula III, 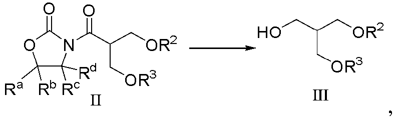 wherein R^{a}, R^{b}, R^{c}, R^{d}, R² and R³ are as defined above.

Further, the step of converting the compound of formula II into the compound of formula III is carried out in the presence of a reducing agent.

Further, in the step of converting the compound of formula II into the compound of formula III, the reducing agent used in the reaction process is sodium borohydride.

In some embodiments, the method for preparing the compound of formula VIII or the salt thereof comprises the step of converting the compound of formula III into a compound of formula IV, 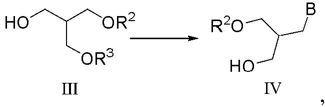 wherein R², R³ and B are as defined above.

Further, in the step of converting the compound of formula III into the compound of formula IV, an activator is also added in the reaction process.

Further, in the step of converting the compound of formula III into the compound of formula IV, the activator used in the reaction process is preferably diisopropyl azodicarboxylate.

In some embodiments, the method for preparing the compound of formula VIII or the salt thereof comprises the steps of converting the compound of formula II into the compound of formula III, and converting the compound of formula III into the compound of formula IV, 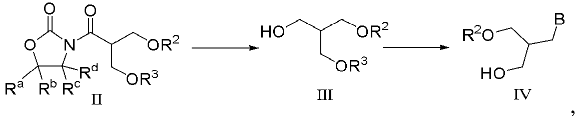 wherein R^{a}, R^{b}, R^{c}, R^{d}, R², R³ and B are as defined above.

Further, in some embodiments, R^{a}, R^{b} and R^{c} are all hydrogen.

Further, in some embodiments, R^{d} is benzyl.

In some embodiments, the method for preparing the compound of formula VIII or the salt thereof comprises the step of converting the compound of formula IV into a compound of formula V, 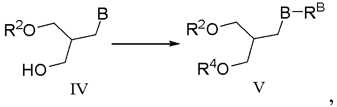 wherein R², B, R⁴ and R^{B} are as defined above.

In some embodiments, the method for preparing the compound of formula VIII or the salt thereof comprises the step of converting the compound of formula V into a compound of formula VI, 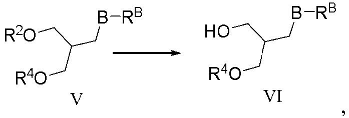 wherein R², B, R⁴ and R^{B} are as defined above.

Further, the step of converting the compound of formula V into the compound of formula VI is reacted in the presence of a reducing agent.

Further, in the step of converting the compound of formula V into the compound of formula VI, the reducing agent used in the reaction process is Pd(OH)₂/C.

In some embodiments, the method for preparing the compound of formula VIII or the salt thereof comprises the steps of converting the compound of formula IV into the compound of formula V, and converting the compound of formula V into the compound of formula VI, 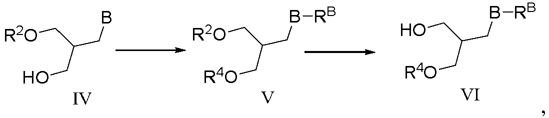 wherein R², B, R⁴ and R^{B} are as defined above.

In some embodiments, the method for preparing the compound of formula VIII or the salt thereof comprises the step of converting the compound of formula VI into the compound of formula VII, 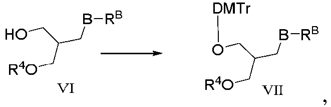 wherein B, R⁴ and R^{B} are as defined above.

In some embodiments, the method for preparing the compound of formula VIII or the salt thereof comprises the step of converting the compound of formula VII into the compound of formula VIII, 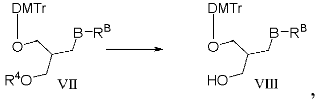 wherein B, R⁴ and R^{B} are as defined above.

In some embodiments, the method for preparing the compound of formula VIII or the salt thereof comprises the steps of converting the compound of formula VI into the compound of formula VII, and converting the compound of formula VII into the compound of formula VIII, 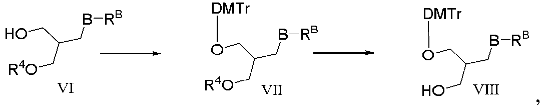 wherein B, R⁴ and R^{B} are as defined above.

In some embodiments, the method for preparing the compound of formula VIII or the salt thereof comprises the steps of
a) converting the compound of formula II into the compound of formula III,
b) converting the compound of formula III into the compound of formula IV,
c) converting the compound of formula IV into the compound of formula V,
d) converting the compound of formula V into the compound of formula VI,
e) reacting the compound of formula VI with 4,4'-dimethoxytrityl halide to form the compound of formula VII, and
f) converting the compound of formula VII into the compound of formula VIII, 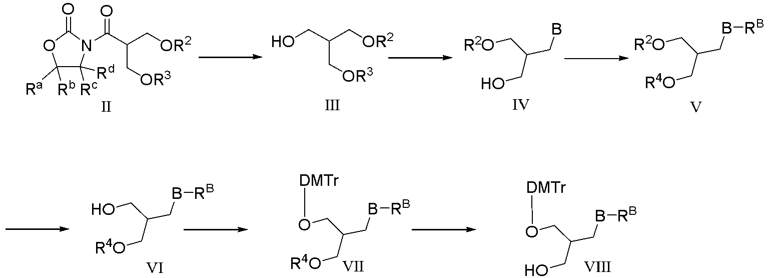
wherein R^{a}, R^{b}, R^{c}, R^{d}, R², R³, R⁴, B and R^{B} are as defined above.

In another aspect, in the method of the disclosure, the compound of formula VIII is a compound of formula VIII-1, 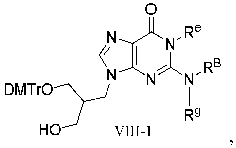 the method comprises the step of converting a compound of formula II-1 into a compound of formula VII-1, 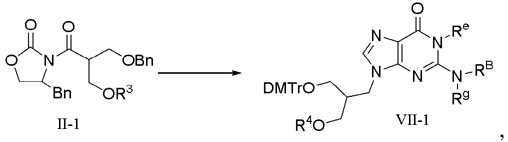 wherein R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl; R³ and R^{B} are as defined above.

In some embodiments, the method for preparing the compound of formula VIII-1 or the salt thereof comprises the step of converting the compound of formula II-1 into a compound of formula III-1, 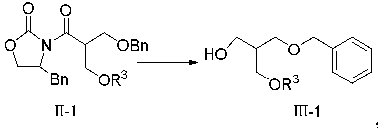 wherein R³ is as defined above.

Further, the step of converting the compound of formula II-1 into the compound of formula III-1 is carried out in the presence of a reducing agent.

Further, in the step of converting the compound of formula II-1 into the compound of formula III-1, the reducing agent used in the reaction process is sodium borohydride.

In some embodiments, the method for preparing the compound of formula VIII-1 or the salt thereof comprises the step of reacting the compound of formula III-1 with a compound of formula D to form a compound of formula IV-1, 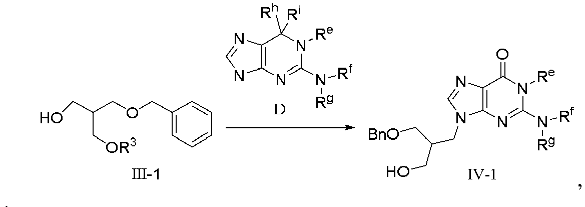 wherein R^{f}, R^{h} and Rⁱ are the same or different, and are each independently selected from hydrogen, halogen, hydroxyl, amino, C₁₋₆ haloalkyl, C₁₋₆ hydroxy-substituted alkyl or C₁₋₆ amino-substituted alkyl; alternatively, R^{h} and Rⁱ together with the carbon atom to which they are attached form an oxo group or a thio group; R^{e}, R^{g} and R³ are as defined above.

Further, in some embodiments, R^{h} is hydrogen, and R is halogen, wherein the halogen is selected from fluorine, chlorine, bromine or iodine, preferably chlorine.

Further, in some embodiments, R^{f} is hydrogen.

Further, in some embodiments, both R^{e} and R^{g} are hydrogen.

Further, in the step of converting the compound of formula III-1 into the compound of formula IV-1, an activator is also added in the reaction process.

Further, in the step of converting the compound of formula III-1 into the compound of formula IV-1, the activator used in the reaction process is preferably diisopropyl azodicarboxylate.

In some embodiments, the method for preparing the compound of formula VIII-1 or the salt thereof comprises the steps of converting the compound of formula II-1 into the compound of formula III-1, and reacting the compound of formula III-1 with the compound of formula D to form the compound of formula IV-1, 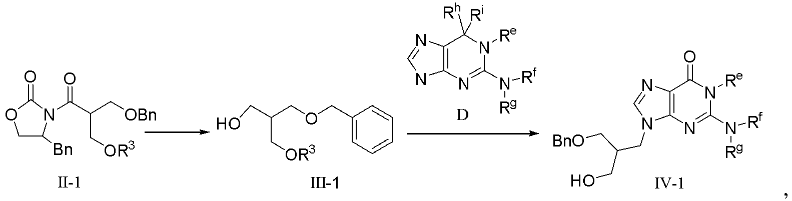 wherein R^{f}, R^{h}, Rⁱ, R^{h}, Rⁱ, R^{e}, R^{g} and R³ are as defined above.

In some embodiments, the method for preparing the compound of formula VIII-1 or the salt thereof comprises the step of reacting the compound of formula IV-1 with an acyl halide compound X¹-R^{B} to form a compound of formula V-1, 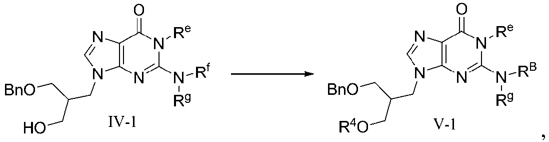 wherein in the acyl halide compound, X¹ is connected to the carbonyl on R^{B}; X¹ represents halogen and is selected from fluorine, chlorine, bromine or iodine, preferably chlorine; R^{e}, R^{f}, R^{g}, R⁴ and R^{B} are as defined above.

In some embodiments, the method for preparing the compound of formula VIII-1 or the salt thereof comprises the step of converting the compound of formula V-1 into a compound of formula VI-1, 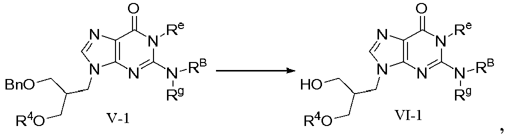 wherein R^{e}, R^{f}, R^{g}, R⁴ and R^{B} are as defined above.

Further, the step of converting the compound of formula V-1 into the compound of formula VI-1 is reacted in the presence of a reducing agent.

Further, in the step of converting the compound of formula V-1 into the compound of formula VI-1, the reducing agent used in the reaction process is Pd(OH)₂/C.

In some embodiments, the method for preparing the compound of formula VIII-1 or the salt thereof comprises the steps of reacting the compound of formula IV-1 with the acyl halide compound X¹-R^{B} to form the compound of formula V-1, and converting the compound of formula V-1 into the compound of formula VI-1, 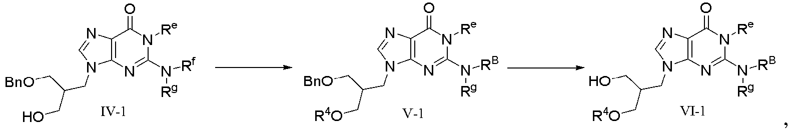 wherein X¹, R^{e}, R^{f}, R^{g}, R⁴ and R^{B} are as defined above.

In some embodiments, the method for preparing the compound of formula VIII-1 or the salt thereof comprises the step of reacting the compound of formula VI-1 with DMTr-X² to form the compound of formula VII-1, 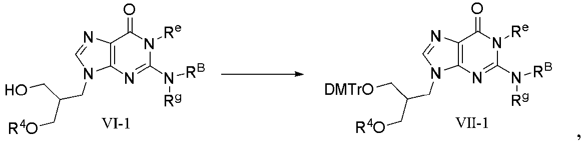 wherein X² represents halogen and is selected from fluorine, chlorine, bromine or iodine, preferably chlorine; R^{e}, R^{g}, R⁴ and R^{B} are as defined above.

In some embodiments, the method for preparing the compound of formula VIII-1 or the salt thereof comprises the step of converting the compound of formula VII-1 into the compound of formula VIII-1, 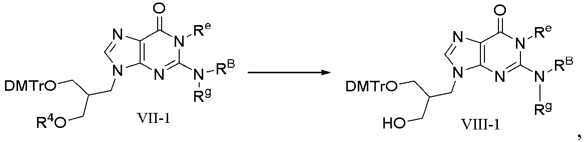 wherein R^{e}, R^{g}, R⁴ and R^{B} are as defined above.

In some embodiments, the method for preparing the compound of formula VIII-1 or the salt thereof comprises the steps of reacting the compound of formula VI-1 with DMTr-X² to form the compound of formula VII-1, and converting the compound of formula VII-1 into the compound of formula VIII-1, 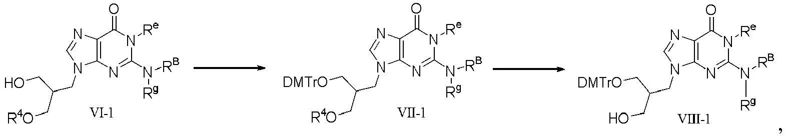 wherein X², R^{e}, R^{g}, R⁴ and R^{B} are as defined above.

In some embodiments, the method for preparing the compound of formula VIII-1 or the salt thereof comprises the steps of:
a) converting the compound of formula II-1 into the compound of formula III-1,
b) reacting the compound of formula III-1 with the compound of formula D to form the compound of formula IV-1,
c) reacting the compound of formula IV-1 with the acyl halide compound X¹-R^{B} to form the compound of formula V-1,
d) converting the compound of formula V-1 into the compound of formula VI-1,
e) reacting the compound of formula VI-1 with DMTr-X² to form the compound of formula VII-1, and
f) converting the compound of formula VII-1 into the compound of formula VIII-1, 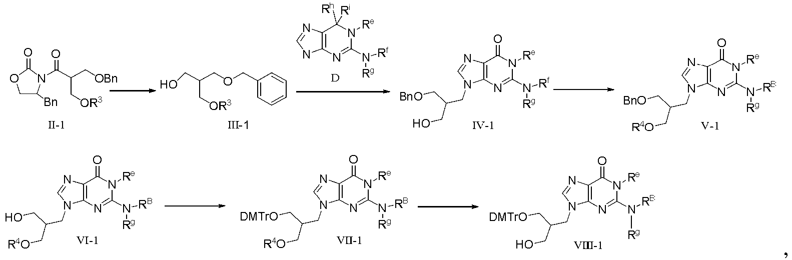 wherein R³, R⁴, R^{B}, R^{e}, R^{f}, R^{g}, R^{h}, Rⁱ, X¹ and X² are as defined above.

In another aspect, in the method of the disclosure, the compound of formula VIII or formula VIII-1 is a compound of formula VIII-1a, 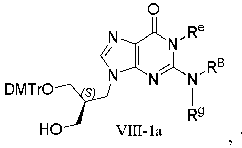 wherein the method comprises the step of converting a compound of formula II-1a into a compound of formula VII-1a, 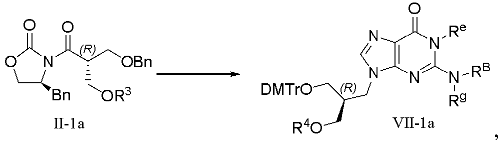 wherein R^{e}, R^{g}, R³ and R^{B} are as defined above.

In some embodiments, the method for preparing the compound of formula VIII-1a or the salt thereof comprises the step of converting a compound of formula II-1b into the compound of formula VII-1a, 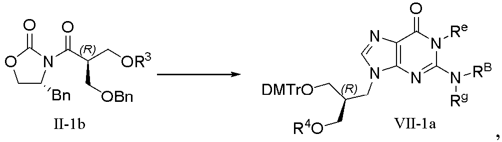 wherein R^{e}, R^{g}, R³ and R^{B} are as defined above.

In some embodiments, the method for preparing the compound of formula VIII-1a or the salt thereof comprises the step of converting the compound of formula II-1a into the compound of formula III-1a, 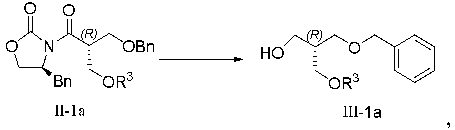 wherein R³ is as defined above.

Further, the step of converting the compound of formula II-1a into the compound of formula III-1a is carried out in the presence of a reducing agent.

Further, in the step of converting the compound of formula II-1a into the compound of formula III-1a, the reducing agent used in the reaction process is sodium borohydride.

In some embodiments, the method for preparing the compound of formula VIII-1a or the salt thereof comprises the step of converting the compound of formula II-1b into a compound of formula III-1b, 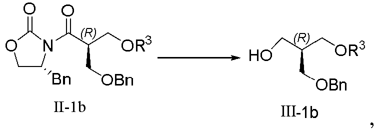 wherein R³ is as defined above.

Further, the step of converting the compound of formula II-1b into the compound of formula III-1b is carried out in the presence of a reducing agent.

Further, in the step of converting the compound of formula II-1b into the compound of formula III-1b, the reducing agent used in the reaction process is sodium borohydride.

In some embodiments, the method for preparing the compound of formula VIII-1a or the salt thereof comprises the step of reacting the compound of formula III-1a with the compound of formula D to form a compound of formula IV-1a, 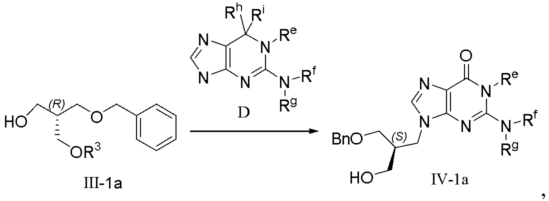 wherein R^{f}, R^{h}, Rⁱ, R^{e}, R^{g} and R³ are as defined above.

Further, in the step of converting the compound of formula III-1a into the compound of formula IV-1a, an activator is also added in the reaction process.

Further, in the step of converting the compound of formula III-1a into the compound of formula IV-1a, the activator used in the reaction process is preferably diisopropyl azodicarboxylate.

In some embodiments, the method for preparing the compound of formula VIII-1a or the salt thereof comprises the step of reacting the compound of formula III-1b with the compound of formula D to form the compound of formula IV-1a, 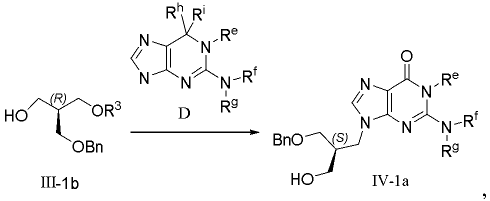 wherein R^{f}, R^{h}, Rⁱ, R^{e}, R^{g} and R³ are as defined above.

Further, in the step of converting the compound of formula III-1b into the compound of formula IV-1a, an activator is also added in the reaction process.

Further, in the step of converting the compound of formula III-1b into the compound of formula IV-1a, the activator used in the reaction process is preferably diisopropyl azodicarboxylate.

In some embodiments, the method for preparing the compound of formula VIII-1a or the salt thereof comprises the steps of converting the compound of formula II-1a into the compound of formula III-1a, and reacting the compound of formula III-1a with the compound of formula D to form the compound of formula IV-1a, 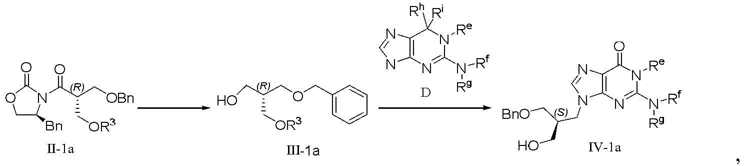 wherein R^{f}, R^{h}, Rⁱ, R^{h}, Rⁱ, R^{e}, R^{g} and R³ are as defined above.

In some embodiments, the method for preparing the compound of formula VIII-1a or the salt thereof comprises the steps of converting the compound of formula II-1b into the compound of formula III-1b, and reacting the compound of formula III-1b with the compound of formula D to form the compound of formula IV-1a, 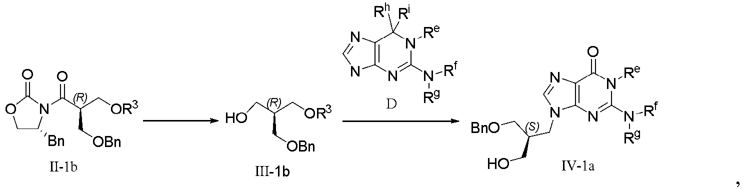 wherein R^{f}, R^{h}, Rⁱ, R^{h}, Rⁱ, R^{e}, R^{g} and R³ are as defined above.

In some embodiments, the method for preparing the compound of formula VIII-1a or the salt thereof comprises the step of reacting the compound of formula IV-1a with the acyl halide compound X¹-R^{B} to form a compound of formula V-1a, 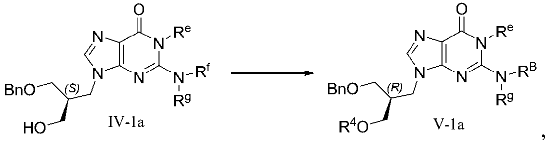 wherein X¹, R^{e}, R^{f}, R^{g}, R⁴ and R^{B} are as defined above.

In some embodiments, the method for preparing the compound of formula VIII-1a or the salt thereof comprises the step of converting the compound of formula V-1a into a compound of formula VI-1a, 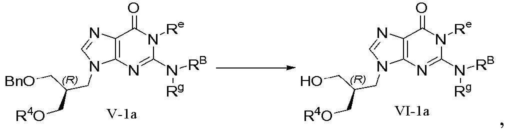 wherein R^{e}, R^{f}, R^{g}, R⁴ and R^{B} are as defined above.

Further, the step of converting the compound of formula V-1a into the compound of formula VI-1a is reacted in the presence of a reducing agent.

Further, in the step of converting the compound of formula V-1a into the compound of formula VI-1a, the reducing agent used in the reaction process is Pd(OH)₂/C.

In some embodiments, the method for preparing the compound of formula VIII-1a or the salt thereof comprises the steps of reacting the compound of formula IV-1a with the acyl halide compound X¹-R^{B} to form the compound of formula V-1a, and converting the compound of formula V-1a into the compound of formula VI-1a, 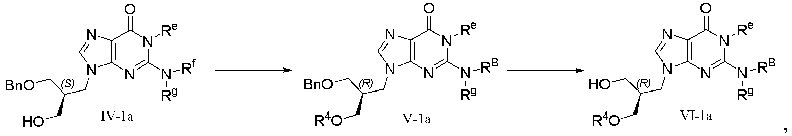 wherein X¹, R^{e}, R^{f}, R^{g}, R⁴ and R^{B} are as defined above.

In some embodiments, the method for preparing the compound of formula VIII-1a or the salt thereof comprises the step of reacting the compound of formula VI-1a with DMTr-X² to form the compound of formula VII-1a, 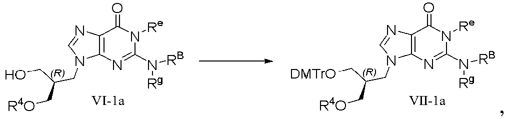 wherein X², R^{e}, R^{g}, R⁴ and R^{B} are as defined above.

In some embodiments, the method for preparing the compound of formula VIII-1a or the salt thereof comprises the step of converting the compound of formula VII-1a into a compound of formula VIII-1a, 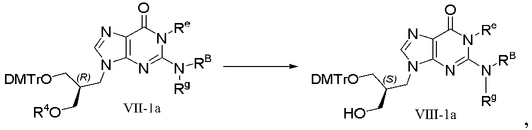 wherein R^{e}, R^{g}, R⁴ and R^{B} are as defined above.

In some embodiments, the method for preparing the compound of formula VIII-1a or the salt thereof comprises the steps of reacting the compound of formula VI-1a with DMTr-X² to form the compound of formula VII-1a, and converting the compound of formula VII-1a into the compound of formula VIII-1a, 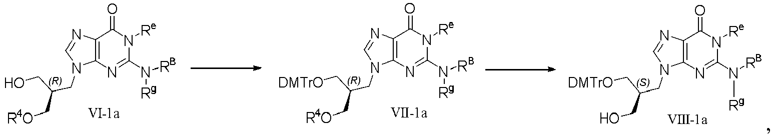 wherein X², R^{e}, R^{g}, R⁴ and R^{B} are as defined above.

In some embodiments, the method for preparing the compound of formula VIII-1a or the salt thereof comprises the steps of:
a) converting the compound of formula II-1a into the compound of formula III-1a,
b) reacting the compound of formula III-1a with the compound of formula D to form the compound of formula IV-1a,
c) reacting the compound of formula IV-1a with the acyl halide compound X¹-R^{B} to form the compound of formula V-1a,
d) converting the compound of formula V-1a into the compound of formula VI-1a,
e) reacting the compound of formula VI-1a with DMTr-X² to form the compound of formula VII-1a, and
f) converting the compound of formula VII-1a into the compound of formula VIII-1a, 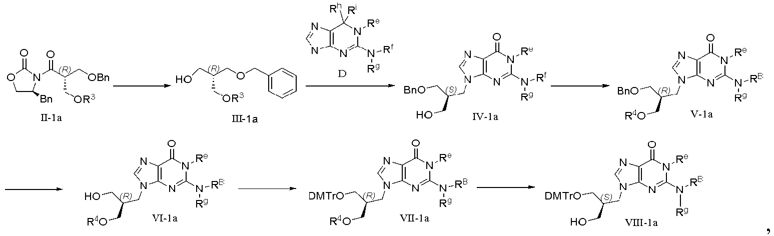
wherein R³, R⁴, R^{B}, R^{e}, R^{f}, R^{g}, R^{h}, Rⁱ, X¹ and X² are as defined above.

In some embodiments, the method for preparing the compound of formula VIII-1a or the salt thereof comprises the steps of:
a) converting the compound of formula II-1b into the compound of formula III-1b,
b) reacting the compound of formula III-1b with the compound of formula D to form the compound of formula IV-1a,
c) reacting the compound of formula IV-1a with the acyl halide compound X¹-R^{B} to form the compound of formula V-1a,
d) converting the compound of formula V-1a into the compound of formula VI-1a,
e) reacting the compound of formula VI-1a with DMTr-X² to form the compound of formula VII-1a, and
f) converting the compound of formula VII-1a into the compound of formula VIII-1a, 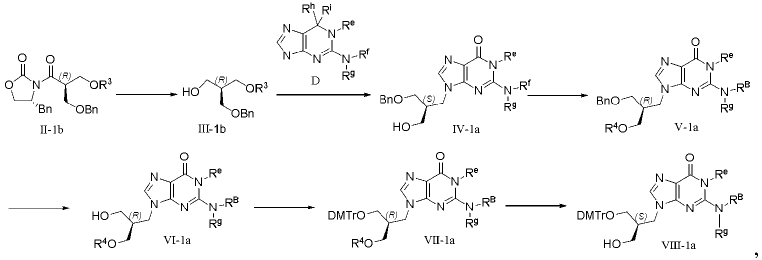 wherein R³, R⁴, R^{B}, R^{e}, R^{f}, R^{g}, R^{h}, Rⁱ, X¹ and X² are as defined above.

In another aspect, in the method of the disclosure, the compound of formula VIII, formula VIII-1 or formula VIII-1a is a compound of formula VIII-2, 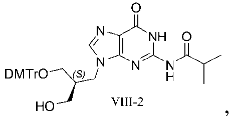 wherein the method comprises the step of converting a compound of formula II-2a into a compound of formula III-2a,

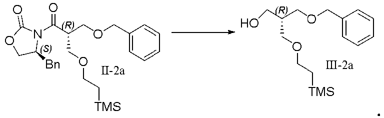

Further, the step of converting the compound of formula II-2a into the compound of formula III-2a is carried out in the presence of a reducing agent.

Further, in the step of converting the compound of formula II-2a into the compound of formula III-2a, the reducing agent used in the reaction process is sodium borohydride.

In some embodiments, the method for preparing the compound of formula VIII-2 or the salt thereof comprises the step of converting a compound of formula II-2b into a compound of formula III-2b,

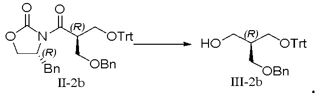

Further, the step of converting the compound of formula II-2b into the compound of formula III-2b is carried out in the presence of a reducing agent.

Further, in the step of converting the compound of formula II-2b into the compound of formula III-2b, the reducing agent used in the reaction process is sodium borohydride.

In some embodiments, the method for preparing the compound of formula VIII-2 or the salt thereof comprises the step of reacting the compound of formula III-2a with a compound of formula 1d to form a compound of formula IV-2,

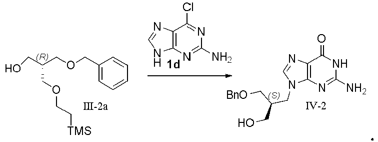

Further, in the step of converting the compound of formula III-2a into the compound of formula IV-2, an activator is also added in the reaction process.

Further, in the step of converting the compound of formula III-2a into the compound of formula IV-2, the activator used in the reaction process is preferably diisopropyl azodicarboxylate.

In some embodiments, the method for preparing the compound of formula VIII-2 or the salt thereof comprises the step of reacting the compound of formula III-2b with the compound of formula 1d to form the compound of formula IV-2,

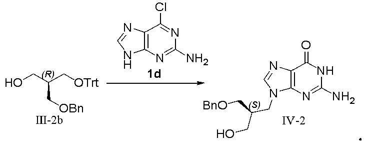

Further, in the step of converting the compound of formula III-2b into the compound of formula IV-2, an activator is also added in the reaction process.

Further, in the step of converting the compound of formula III-2b into the compound of formula IV-2, the activator used in the reaction process is preferably diisopropyl azodicarboxylate.

In some embodiments, the method for preparing the compound of formula VIII-2 or the salt thereof comprises the step of reacting the compound of formula IV-2 with X¹-C(O)-CH(CH₃)₂ to form a compound of formula V-2, wherein in the step, 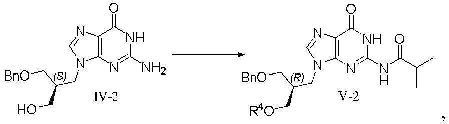 R⁴ and X¹ are as defined above.

Further, in the step of reacting the compound of formula IV-2 with X¹-C(O)-CH(CH₃)₂ to form the compound of formula V-2, TBS is used as a hydroxyl protecting group.

Further, in the step of reacting the compound of formula IV-2 with X¹-C(O)-CH(CH₃)₂ to form the compound of formula V-2, TBDPS is used as a hydroxyl protecting group.

In some embodiments, the method for preparing the compound of formula VIII-2 or the salt thereof comprises the step of converting the compound of formula V-2 into a compound of formula VI-2, 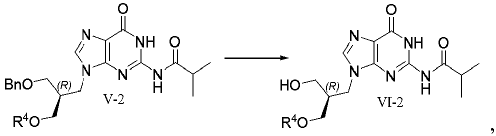 wherein R⁴ is as defined above.

Further, the step of converting the compound of formula V-2 into the compound of formula VI-2 is reacted in the presence of a reducing agent.

Further, in the step of converting the compound of formula V-2 into the compound of formula VI-2, the reducing agent used in the reaction process is Pd(OH)₂/C.

In some embodiments, the method for preparing the compound of formula VIII-2 or the salt thereof comprises the step of reacting the compound of formula VI-2 with DMTr-X² to form a compound of formula VII-2, 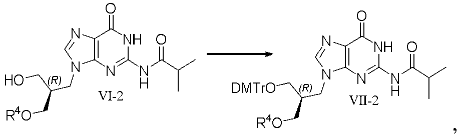 wherein R⁴ and X² are as defined above.

In some embodiments, the method for preparing the compound of formula VIII-2 or the salt thereof comprises the step of converting the compound of formula VII-2 into the compound of formula VIII-2, 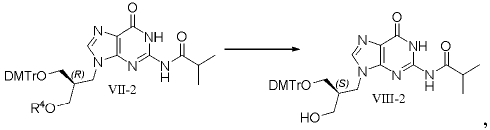 wherein R⁴ is as defined above.

In some embodiments, the method for preparing the compound of formula VIII-2 or the salt thereof comprises the steps of:
a) converting the compound of formula II-2a into the compound of formula III-2a,
b) reacting the compound of formula III-2a with the compound of formula D to form the compound of formula IV-2,
c) reacting the compound of formula IV-2 with the acyl halide compound X¹-R^{B} to form the compound of formula V-2,
d) converting the compound of formula V-2 into the compound of formula VI-2,
e) reacting the compound of formula VI-2 with DMTr-X² to form the compound of formula VII-2, and
f) converting the compound of formula VII-2 into the compound of formula VIII-2, 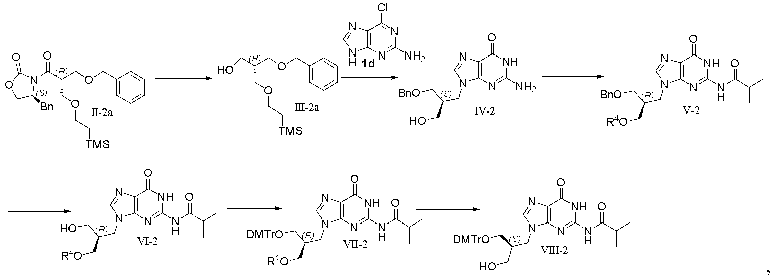 wherein R⁴, X¹ and X² are as defined above.

In some embodiments, the method for preparing the compound of formula VIII-2 or the salt thereof comprises the steps of:
a) converting the compound of formula II-2b into the compound of formula III-2b,
b) reacting the compound of formula III-2b with the compound of formula D to form the compound of formula IV-2,
c) reacting the compound of formula IV-2 with the acyl halide compound X¹-R^{B} to form the compound of formula V-2,
d) converting the compound of formula V-2 into the compound of formula VI-2,
e) reacting the compound of formula VI-2 with DMTr-X² to form the compound of formula VII-2, and
f) converting the compound of formula VII-2 into the compound of formula VIII-2, 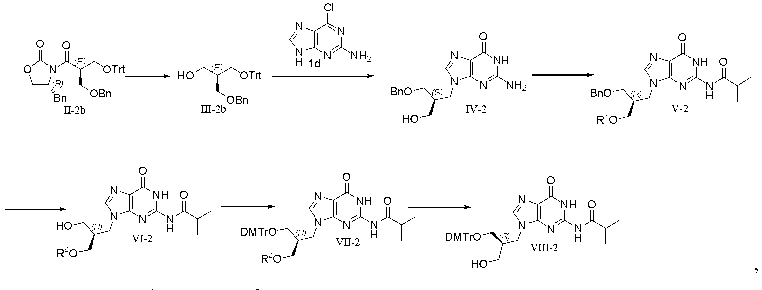 wherein R⁴, X¹ and X² are as defined above.

In another aspect, the disclosure further provides a method for preparing the compound of formula II, wherein the method comprises the step of converting a compound of formula I-1 into the compound of formula II, 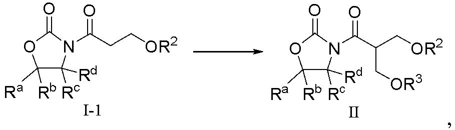 wherein R^{a}, R^{b} and R^{c} are the same or different, and are each independently selected from hydrogen, halogen, hydroxyl, amino, C₁₋₆ alkyl, C₁₋₆ alkoxy, 3- to 10-membered cycloalkyl, 3- to 10-membered heterocyclyl, 6- to 10-membered aryl or 5- to 10-membered heteroaryl, wherein the alkyl, alkoxy, cycloalkyl, heterocyclyl, aryl and heteroaryl are optionally substituted with one or more substituents selected from halogen, hydroxyl, amino, C₁₋₆ alkyl or C₁₋₆ alkoxy; R^{d} is selected from benzyl, phenyl, biphenyl or C₁₋₆ alkyl, wherein the benzyl, phenyl, biphenyl and C₁₋₆ alkyl are optionally substituted with one or more substituents selected from halogen, hydroxyl, amino, C₁₋₆ alkyl or C₁₋₆ alkoxy; R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl; R³ is selected from trityl, tert-butyldimethylsilyl, tert-butyldiphenylsilyl, (trimethylsilyl)ethyl or 2-tetrahydropyran, preferably trimethylsilyl or trityl.

In some embodiments, the step of converting the compound of formula I-1 into the compound of formula II is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In some embodiments, in the method of the disclosure, the compound of formula II is the compound of formula II-1, wherein the method comprises the step of converting a compound of formula I-1a into the compound of formula II-1, 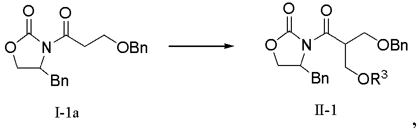 wherein R³ is as defined above. In some embodiments, the step of converting the compound of formula I-1a into the compound of formula II-1 is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In some embodiments, in the method of the disclosure, the compound of formula II or formula II-1 is the compound of formula II-1a, wherein the method comprises the step of converting a compound of formula I-1aa into the compound of formula II-1a, 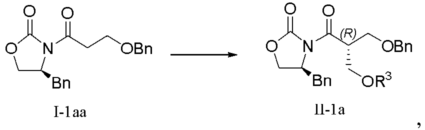 wherein R³ is as defined above. In some embodiments, the step of converting the compound of formula I-1aa into the compound of formula II-1a is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In some embodiments, in the method of the disclosure, the compound of formula II, formula II-1 or formula II-1a is the compound of formula II-2a, wherein the method comprises the step of reacting the compound of formula I-1aa with SEMCl to form the compound of formula II-2a, 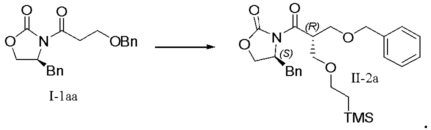 In some embodiments, the step of reacting the compound of formula I-1aa with SEMCl to form the compound of formula II-2a is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In another aspect, the disclosure further provides a method for preparing the compound of formula II, wherein the method comprises the step of converting a compound of formula I-2 into the compound of formula II, 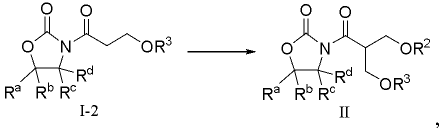 wherein R^{a}, R^{b} and R^{c} are the same or different, and are each independently selected from hydrogen, halogen, hydroxyl, amino, C₁₋₆ alkyl, C₁₋₆ alkoxy, 3- to 10-membered cycloalkyl, 3- to 10-membered heterocyclyl, 6- to 10-membered aryl or 5- to 10-membered heteroaryl, wherein the alkyl, alkoxy, cycloalkyl, heterocyclyl, aryl and heteroaryl are optionally substituted with one or more substituents selected from halogen, hydroxyl, amino, C₁₋₆ alkyl or C₁₋₆ alkoxy; R^{d} is selected from benzyl, phenyl, biphenyl or C₁₋₆ alkyl, wherein the benzyl, phenyl, biphenyl and C₁₋₆ alkyl are optionally substituted with one or more substituents selected from halogen, hydroxyl, amino, C₁₋₆ alkyl or C₁₋₆ alkoxy; R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl; R³ is selected from trityl, tert-butyldimethylsilyl, tert-butyldiphenylsilyl, (trimethylsilyl)ethyl or 2-tetrahydropyran, preferably trimethylsilyl or trityl. In some embodiments, the step of converting the compound of formula I-2 into the compound of formula II is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In some embodiments, in the method of the disclosure, the compound of formula II is the compound of formula II-1, wherein the method comprises the step of converting a compound of formula I-2a into the compound of formula II-1a, 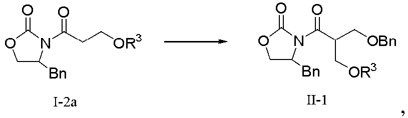 wherein R³ is as defined above. In some embodiments, the step of converting the compound of formula I-2a into the compound of formula II-1a is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In some embodiments, in the method of the disclosure, the compound of formula II or formula II-1 is the compound of formula II-1b, wherein the method comprises the step of converting a compound of formula I-2aa into the compound of formula II-1a, 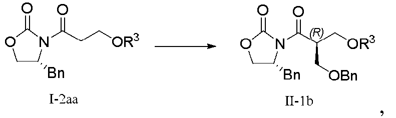 wherein R³ is as defined above. In some embodiments, the step of converting the compound of formula I-2aa into the compound of formula II-1a is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In some embodiments, in the method of the disclosure, the compound of formula II, formula II-1 or formula II-1b is the compound of formula II-2b, wherein the method comprises the step of reacting a compound of formula I-1b with BOMCl to form the compound of formula II-1a, 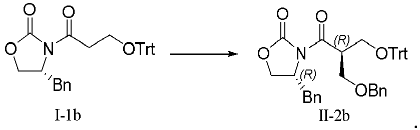 In some embodiments, the step of reacting the compound of formula I-1b with BOMCl to form the compound of formula II-1a is carried out under an organic base condition. In some embodiments, the organic base is selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably lithium bis(trimethylsilyl)amide.

In another aspect, the disclosure further provides a method for preparing the compound of formula I-1aa, wherein the method comprises the steps of converting a compound of formula A into a compound of formula B-1, and reacting the compound of formula B-1 with a compound of formula C-1 to form the compound of formula I-1aa,

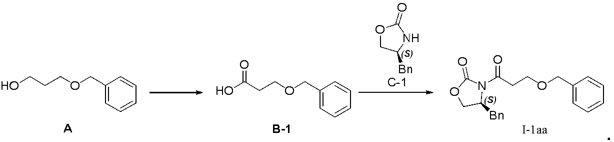

In some embodiments, the step of converting the compound of formula A into the compound of formula B-1 is carried out under a TEMPO catalysis condition. Further, in the step of converting the compound of formula A into the compound of formula B-1, NaClO₂ and NaClO are sequentially added.

In some embodiments, the step of reacting the compound of formula B-1 with the compound of formula C-1 to form the compound of formula I-1aa further comprises the steps of reacting the compound of formula B-1 with pivaloyl chloride to form an acyl chloride intermediate, and reacting the obtained acyl chloride intermediate with the compound of formula C-1 under the catalysis of lithium salt to form the compound of formula I-1aa.

Further, in the step of reacting the compound of formula B-1 with pivaloyl chloride to form the acyl chloride intermediate, an activator is also added. The activator is an organic base selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably triethylamine.

Further, in the step of reacting the obtained acyl chloride intermediate with the compound of formula C-1 under the catalysis of lithium salt to form the compound of formula I-1aa, the lithium salt is lithium chloride.

In another aspect, the disclosure further provides a method for preparing the compound of formula I-1aa, wherein the method comprises the step of reacting a compound of formula B-2 with a compound of formula C-2 to form the compound of formula I-1b,

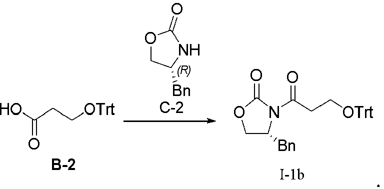

In some embodiments, the step of reacting the compound of formula B-2 with the compound of formula C-2 to form the compound of formula I-1b further comprises the steps of reacting the compound of formula B-2 with pivaloyl chloride to form an acyl chloride intermediate, and reacting the obtained acyl chloride intermediate with the compound of formula C-2 under the catalysis of lithium salt to form the compound of formula I-1b.

Further, in the step of reacting the compound of formula B-2 with pivaloyl chloride to form the acyl chloride intermediate, an activator is also added. The activator is an organic base selected from triethylamine, N, N-diisopropylethylamine, n-butyllithium, lithium diisopropylamide, lithium bis(trimethylsilyl)amide, potassium acetate, sodium tert-butoxide, potassium tert-butoxide and sodium n-butoxide, preferably triethylamine.

Further, in the step of reacting the obtained acyl chloride intermediate with the compound of formula C-2 under the catalysis of lithium salt to form the compound of formula I-1b, the lithium salt is lithium chloride.

In another aspect, the disclosure further provides a compound represented by formula I-2a or a pharmaceutically acceptable salt thereof, 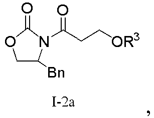 wherein R³ is selected from trityl, tert-butyldimethylsilyl, tert-butyldiphenylsilyl, (trimethylsilyl)ethyl or 2-tetrahydropyran, preferably trimethylsilyl or trityl.

In some embodiments, the compound represented by formula I-2a is a compound represented by formula I-2aa, 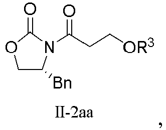 wherein R³ is as defined above.

In some embodiments, the compound represented by formula I-2a or formula I-2aa is a compound represented by formula I-1b,

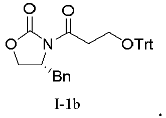

In another aspect, the disclosure further provides a compound represented by formula II or a pharmaceutically acceptable salt thereof, 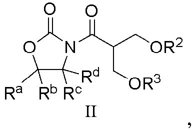 wherein
R^{a}, R^{b} and R^{c} are the same or different, and are each independently selected from hydrogen, halogen, hydroxyl, amino, C₁₋₆ alkyl, C₁₋₆ alkoxy, 3- to 10-membered cycloalkyl, 3- to 10-membered heterocyclyl, 6- to 10-membered aryl or 5- to 10-membered heteroaryl, wherein the alkyl, alkoxy, cycloalkyl, heterocyclyl, aryl and heteroaryl are optionally substituted with one or more substituents selected from halogen, hydroxyl, amino, C₁₋₆ alkyl or C₁₋₆ alkoxy;
R^{d} is selected from benzyl, phenyl, biphenyl or C₁₋₆ alkyl, wherein the benzyl, phenyl, biphenyl and C₁₋₆ alkyl are optionally substituted with one or more substituents selected from halogen, hydroxyl, amino, C₁₋₆ alkyl or C₁₋₆ alkoxy;
R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl;
R³ is selected from trityl, tert-butyldimethylsilyl, tert-butyldiphenylsilyl, (trimethylsilyl)ethyl or 2-tetrahydropyran, preferably trimethylsilyl or trityl.

In some embodiments, the compound represented by formula II is a compound represented by formula II-1, 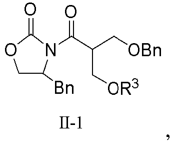 wherein R³ is as defined above.

In some embodiments, the compound represented by formula II or formula II-1 is a compound represented by formula II-1a, 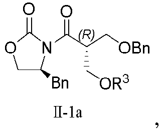 wherein R³ is as defined above.

In some embodiments, the compound represented by formula II or formula II-1 is a compound represented by formula II-1b, 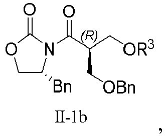 wherein R³ is as defined above.

In some embodiments, the compound represented by formula II, formula II-1 or formula II-1a is a compound represented by formula II-2a,

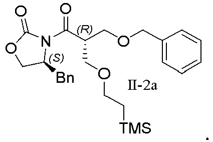

In some embodiments, the compound represented by formula II, formula II-1 or formula II-1b is a compound represented by formula II-2b,

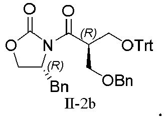

In another aspect, the disclosure further provides a compound represented by formula III or a pharmaceutically acceptable salt thereof, 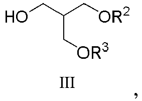 wherein
R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl;
R³ is selected from trityl, tert-butyldimethylsilyl, tert-butyldiphenylsilyl, (trimethylsilyl)ethyl or 2-tetrahydropyran, preferably trimethylsilyl or trityl.

In some embodiments, the compound represented by formula III is a compound represented by formula III-1, 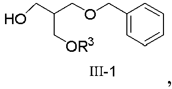 wherein R³ is as defined above.

In some embodiments, the compound represented by formula III or formula III-1 is a compound represented by formula III-1a, 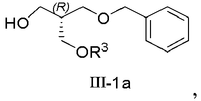 wherein R³ is as defined above.

In some embodiments, the compound represented by formula III or formula III-1 is a compound represented by formula III-1b, 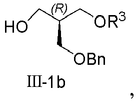 wherein R³ is selected from trityl, tert-butyldimethylsilyl, tert-butyldiphenylsilyl, (trimethylsilyl)ethyl or 2-tetrahydropyran, preferably trimethylsilyl or trityl.

In some embodiments, the compound represented by formula III, formula III-1 or formula III-1a is a compound represented by formula III-2a,

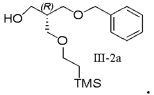

In some embodiments, the compound represented by formula III, formula III-1 or formula III-1b is a compound represented by formula III-2b,

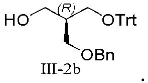

In another aspect, the disclosure further provides a compound represented by formula IV or a pharmaceutically acceptable salt thereof, 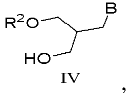 wherein
R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl;
B is a base.

In some embodiments, the compound represented by formula IV is a compound represented by formula IV-1, 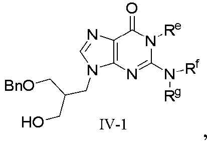 wherein R^{e}, R^{f} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl.

In some embodiments, the compound represented by formula IV or formula IV-1 is a compound represented by formula IV-1a, 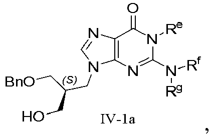 wherein R^{e}, R^{f} and R^{g} are as defined above.

In some embodiments, the compound represented by formula IV, formula IV-1 or formula IV-1a is a compound represented by formula IV-2,

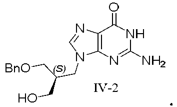

In another aspect, the disclosure further provides a compound represented by formula V or a pharmaceutically acceptable salt thereof, 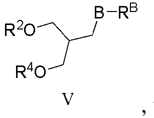 wherein
R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl;
R⁴ is a hydroxyl protecting group, preferably trityl, tert-butyldimethylsilyl, tert-butyldiphenylsilyl, (trimethylsilyl)ethyl or 2-tetrahydropyran, and most preferably tert-butyldimethylsilyl or tert-butyldiphenylsilyl;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl, preferably -C(O)-CH₃, -C(O)-CH₂CH₃ or -C(O)-CH(CH₃)₂, and most preferably -C(O)-CH(CH₃)₂.

In some embodiments, the compound represented by formula V is a compound represented by formula V-1 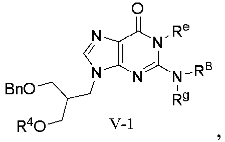 wherein R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl; R⁴ and R^{B} are as defined above.

In some embodiments, the compound represented by formula V or formula V-1 is a compound represented by formula V-1a, 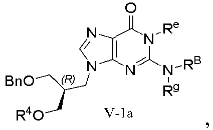 wherein R^{e}, R^{g}, R⁴ and R^{B} are as defined above.

In some embodiments, the compound represented by formula V, formula V-1 or formula V-1a is a compound represented by formula V-2,

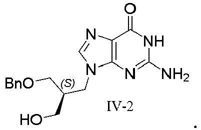

In another aspect, the disclosure further provides a compound represented by formula VI or a pharmaceutically acceptable salt thereof, 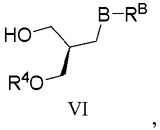 wherein
R⁴ is a hydroxyl protecting group, preferably trityl, tert-butyldimethylsilyl, tert-butyldiphenylsilyl, (trimethylsilyl)ethyl or 2-tetrahydropyran, and most preferably tert-butyldimethylsilyl or tert-butyldiphenylsilyl;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl, preferably -C(O)-CH₃, -C(O)-CH₂CH₃ or -C(O)-CH(CH₃)₂, and most preferably -C(O)-CH(CH₃)₂.

In some embodiments, the compound represented by formula VI is a compound represented by formula VI-1, 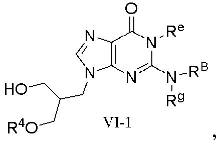 wherein
R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl;
R⁴ and R^{B} are as defined above.

In some embodiments, the compound represented by formula VI or formula VI-1 is a compound represented by formula VI-1a, 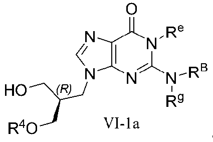 wherein R^{e}, R^{g}, R⁴ and R^{B} are as defined above.

In some embodiments, the compound represented by formula VI, formula VI-1 or formula VI-1a is a compound represented by formula VI-2, 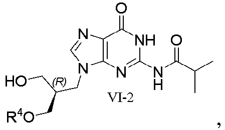 wherein R⁴ is as defined above.

In another aspect, the disclosure further provides a compound represented by formula VII or a pharmaceutically acceptable salt thereof 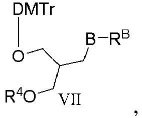 wherein
R⁴ is a hydroxyl protecting group, preferably trityl, tert-butyldimethylsilyl, tert-butyldiphenylsilyl, (trimethylsilyl)ethyl or 2-tetrahydropyran, and most preferably tert-butyldimethylsilyl or tert-butyldiphenylsilyl;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl, preferably -C(O)-CH₃, -C(O)-CH₂CH₃ or -C(O)-CH(CH₃)₂, and most preferably -C(O)-CH(CH₃)₂.

In some embodiments, the compound represented by formula VII is a compound represented by formula VII-1, 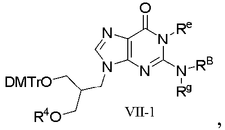 wherein
R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl;
R⁴ and B are as defined above.

In some embodiments, the compound represented by formula VII or formula VII-1 is a compound represented by formula VII-1a, 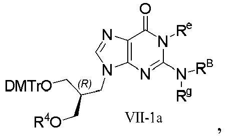 wherein R^{e}, R^{g}, R⁴ and B are as defined above.

In some embodiments, the compound represented by formula VII, formula VII-1 or formula VII-1a is a compound represented by formula VII-2, 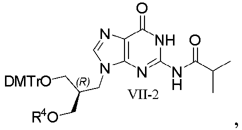 wherein R⁴ is as defined above.

The disclosure further provides the use of the aforementioned formula VIII, formula VIII-1, formula VIII-1a or formula VIII-2 in the preparation of an oligonucleotide analogue, such as the use in the preparation of an siRNA.

The disclosure further provides the use of the aforementioned compound represented by formula II, formula II-1, formula II-1a, formula II-2a, formula II-1b, formula II-2b, formula III, formula III-1, formula III-1a, formula III-2a, formula III-1b, formula III-2b, formula IV, formula IV-1, formula IV-1a, formula IV-2, formula V, formula V-1, formula V-1a, formula V-2, formula VI, formula VI-1, formula VI-1a, formula VI-2, formula VII, formula VII-1, formula VII-1a or formula VII-2 in the preparation of an oligonucleotide analogue, such as the use in the preparation of an siRNA.

Further, the oligonucleotide analogue of the disclosure is selected from but not limited to

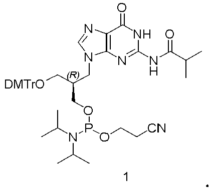

The disclosure further provides a method for preparing compound 1, wherein the method comprises the aforementioned method for preparing the compound represented by formula VIII-2,

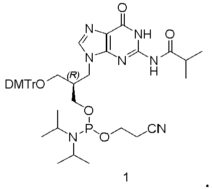

The disclosure further provides a method for preparing compound 1, wherein the method comprises the step of reacting the compound of formula VIII-2 with a compound of formula E to form the compound 1,

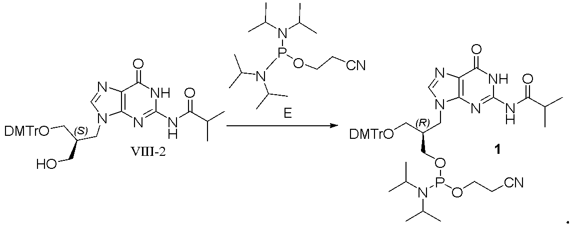

In another aspect, the preparation method described in the disclosure further comprises one or more steps of filtration, concentration, purification by column chromatography and drying.

The terms "to form" and "converting into" do not specifically mean that the conversion reaction between two substrates is a single step, and can be a single step or multi-step reaction between two substrates. If the intermediate contains a protecting group, the intermediate is subjected to a step of removing the protecting agent, and then reacted with a corresponding substrate to obtain a corresponding target product.

The numerical values of the disclosure are instrument measurements and have a certain degree of error. In general, plus or minus 10% is within a reasonable error range. Of course, the context in which the numerical value is used needs to be considered. For example, the error of the numerical value for the particle size of the active ingredient after measurement does not exceed plus or minus 10%, and can does not exceed plus or minus 9%, plus or minus 8%, plus or minus 7%, plus or minus 6%, plus or minus 5%, plus or minus 4%, plus or minus 3%, plus or minus 2% or plus or minus 1%, preferably plus or minus 5%.

The technical solutions of the disclosure have the following beneficial effects: (1) simple process steps and reduced production costs; (2) high reaction yield and high purity; and (3) no additional chiral resolution step, making it suitable for large-scale production.

Unless stated to the contrary, the terms used in the description and claims have the following meanings.

The pharmaceutically acceptable salt of the compound described in the disclosure may be selected from an inorganic salt or an organic salt.

The compounds of the disclosure may exist in specific geometric or stereoisomeric forms. The disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, and all such mixtures fall within the scope of the disclosure. Additional asymmetric carbon atoms may be present in a substituent such as an alkyl group. All such isomers, as well as mixtures thereof, are included within the scope of the disclosure. The compound containing asymmetric carbon atoms of the disclosure can be isolated in optically active-pure or racemic forms. The optically active-pure form can be resolved from the racemic mixture or synthesized by utilizing chiral raw materials or chiral reagents.

Optically active (R)- and (S)-isomers and D and L isomers can be prepared using chiral synthesis or chiral reagents or other conventional techniques. If a particular enantiomer of a compound of the disclosure is desired, it can be prepared by asymmetric synthesis or derivatization with a chiral auxiliary, wherein the resulting diastereomeric mixture is separated and the auxiliary groups are cleaved to provide the pure desired enantiomer. Alternatively, when the molecule contains a basic functional group (such as an amino group) or an acidic functional group (such as a carboxyl group), diastereomeric salts can be formed with an appropriate optically active acid or base, and then the diastereomers are resolved by conventional methods well known in the art, and the pure enantiomers are recovered. In addition, separation of enantiomers and diastereomers is frequently accomplished by using chromatography using chiral stationary phases, optionally in combination with chemical derivatization methods (e.g., formation of carbamates from amines).

In the chemical structures of the compounds described in the disclosure, the bond " 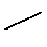 " indicates that the configuration is not specified, i.e. if chiral isomers exist in the chemical structure, the bond " 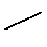 " can be " 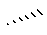 " or " 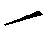 ", or comprises both " 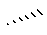 " and " 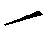 " configurations.

In the chemical structures of the compounds described in the disclosure, the bond " 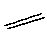 " indicates that the configuration is not specified, that is, it can be a Z configuration or E configuration, or comprises the two configurations.

The compounds and intermediates of the disclosure may also exist in different tautomeric forms, and all such forms are encompassed within the scope of the disclosure. The term "tautomer" or "tautomeric form" refers to structural isomers of different energies which are interconvertible via a low energy barrier. For example, proton tautomers (also known as prototropic tautomers) involve interconversions via proton transfer. For example, the compound of the disclosure involves the tautomeric change between A and B as shown below.

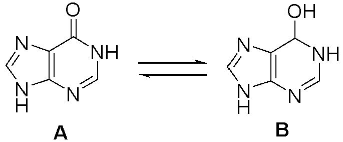

All compounds in the disclosure can be drawn as either form A or form B. All tautomeric forms are included within the scope of the disclosure. The naming of the compound does not exclude any tautomers.

The disclosure also includes some isotopically-labelled compounds of the disclosure which are identical to those recited herein, but have one or more atoms replaced by an atom having an atomic mass or mass number different from the atomic mass or mass number usually found in nature. Examples of isotopes that can be incorporated into the compound of the disclosure include isotopes of hydrogen, carbon, nitrogen, oxygen, phosphorus, sulphur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

Unless otherwise indicated, when a position is specifically designated as deuterium (D), the position should be understood to have an abundance of deuterium at least 1000 times greater than the natural abundance of deuterium (which is 0.015%) (i.e., at least 10% deuterium incorporation). The compound in examples can have an abundance of deuterium at least 1000 times greater than the natural abundance of deuterium, an abundance of deuterium at least 2000 times greater than the natural abundance of deuterium, an abundance of deuterium at least 3000 times greater than the natural abundance of deuterium, an abundance of deuterium at least 4000 times greater than the natural abundance of deuterium, an abundance of deuterium at least 5000 times greater than the natural abundance of deuterium, an abundance of deuterium at least 6000 times greater than the natural abundance of deuterium, or higher abundance of deuterium. The disclosure further includes various deuterated forms of the compound of formula (I). Each available hydrogen atom attached to a carbon atom can be independently replaced by a deuterium atom. Those skilled in the art can synthesize deuterated forms of the compound of formula (I) by referring to relevant literatures. The deuterated forms of the compound of formula (I) can be prepared using commercially available deuterated starting materials, or can be synthesized using deuterated reagents using conventional techniques. The deuterated reagents include, but are not limited to, deuterated borane, a solution of trideuterated borane in tetrahydrofuran, deuterated lithium aluminium hydride, deuterated iodoethane and deuterated iodomethane.

"Optionally" or "optional" means that the event or circumstance subsequently described may but need not to occur, and the description includes the occasion where the event or circumstance occurs or does not occur. For example, "C₁₋₆ alkyl optionally substituted with halogen or cyano" means that halogen or cyano may but need not be present, and the description includes the case where the alkyl group is substituted with halogen or cyano and the case where the alkyl group is not substituted with halogen or cyano.

Explanation of terms:
"Pharmaceutical composition" denotes a mixture containing one or more compounds described herein or physiologically or pharmaceutically acceptable salts or prodrug thereof and other chemical components, as well as other components, such as physiologically or pharmaceutically acceptable carriers and excipients. The purpose of pharmaceutical compositions is to facilitate administration to living organisms and facilitate the absorption of active ingredients to exert biological activity.

The "pharmaceutically acceptable excipient" includes, but is not limited to, any adjuvant, carrier, excipient, glidant, sweetener, diluent, preservative, dye/colorant, flavouring agent, surfactant, wetting agent, dispersant, suspending agent, stabilizer, isotonic agent, solvent, or emulsifier that has been approved by the US Food and Drug Administration to be acceptable for human or livestock use.

The term "effective amount" or "therapeutically effective amount" described in the disclosure includes an amount sufficient to ameliorate or prevent symptoms of a medical condition or the condition. An effective amount also means an amount sufficient to allow or facilitate diagnosis. The effective amount for a particular patient or veterinary subject may vary depending on the following factors: for example, the condition to be treated, the patient's general health, the method, route and dosage of administration, and the severity of side effects. An effective amount may be the maximum dosage or dosage regimen that avoids significant side effects or toxic effects.

The term "alkyl" refers to a saturated aliphatic hydrocarbon group, including straight and branched chain groups comprising 1 to 20 carbon atoms, such as alkyl comprising 1 to 6 carbon atoms. Non-limiting examples of alkyl include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, and the like. The alkyl may be substituted or unsubstituted, and when it is substituted, the substituent may be substituted at any available point of attachment, and is preferably one or more groups independently selected from halogen, hydroxyl, oxo, amino, C₁₋₆ alkyl, C₁₋₆ alkoxy, 3- to 6-membered cycloalkyl, 3- to 6-membered heterocyclyl, 6- to 10-membered aryl or 5- to 10-membered heteroaryl, wherein the alkyl, alkoxy, cycloalkyl, heterocyclyl, aryl or heteroaryl is optionally substituted with one or more halogen, hydroxyl, amino, C₁₋₆ alkyl or C₁₋₆ alkoxy.

The term "alkoxy" refers to -O-(alkyl), wherein the alkyl is as defined above. Non-limiting examples of alkoxy include: methoxy, ethoxy, propoxy, butoxy, cyclopropoxy, cyclobutoxy, cyclopentoxy, and cyclohexoxy. The alkoxy may be optionally substituted or unsubstituted, and when it is substituted, the substituent is preferably one or more groups independently selected from halogen, hydroxyl, oxo, nitro, cyano, amino, C₁₋₆ alkyl, C₁₋₆ alkoxy, 3- to 6-membered cycloalkyl, or 3- to 6-membered heterocyclyl, wherein the alkyl, alkoxy, cycloalkyl or heterocyclyl is substituted with one or more substituents selected from halogen, hydroxyl, amino, C₁₋₆ alkyl or C₁₋₆ alkoxy.

The term "hydroxy" refers to -OH group.

The term "halogen" refers to fluorine, chlorine, bromine or iodine.

The term "cyano" refers to -CN.

The term "amino" refers to -NH₂.

The term "nitro" refers to -NO₂.

The term "oxo" refers to =O substituent.

The term "substituted" refers to one or more hydrogen atoms in the group, preferably at most 5, more preferably 1-3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort.

The term "hydroxyl protecting group" refers to a group that prevents or inhibits the hydroxy group from participating in next reaction until the protecting group is removed. Examples of the hydroxyl protecting group include acetyl, allyl, benzoyl, benzyl, β-methoxyethoxymethyl, methoxymethyl, dimethoxytrityl[di-(4-methoxyphenyl)phenylmethyl], methoxytriphenyl[(4-methoxyphenyl)diphenylmethyl], p-methoxybenzyl ether, methylthiomethyl, trimethylacetyl, tetrahydropyranyl, trityl, silyl (such as: trimethylsilyl, tert-butyldimethylsilyl, tert-butyldiphenylsilyl, triisopropylsiloxymethyl and triisopropylsilyl). Other examples of the hydroxyl protecting group include alkyl groups, such as methyl and tert-butyl, and other ethers, such as ethoxyethyl.

The term "TMS" refers to trimethylsilyl, and the term "TMSCl" refers to trimethylsilyl chloride.

The term "SEM" refers to 2-(trimethylsilyl)ethoxymethyl, and the term "SEMCl" refers to 2-(trimethylsilyl)ethoxymethyl chloride.

The term "TBDPS" refers to tert-butyldiphenyl, and the term "TBDPSCl" refers to tert-butyldiphenylsilyl chloride.

The term "DMTr" refers to 4,4'-dimethoxytrityl, and the term "DMTrCl" refers to 4,4'-dimethoxytrityl chloride.

The term "TBS" refers to tert-butyldimethylsilyl, and the term "TBSCl" refers to tert-butyldimethylsilyl chloride.

The term "Trt" refers to trityl.

The term "LiHMDS" refers to lithium bis(trimethylsilyl)amide.

The term "MTBE" refers to methyl tert-butyl ether.

The term "TBAF" refers to tetrabutylammonium fluoride.

The term "TEMPO" refers to 2,2,6,6-tetramethylpiperidoxyl.

The term "BOM" refers to benzyloxymethyl, and the term "BOMCl" refers to benzyloxymethyl chloride.

The term "DIAD" refers to diisopropyl azodicarboxylate.

The term "base" refers to a natural or unnatural base, which is optionally substituted with a substituent selected from the group consisting of: halogen, hydroxyl, amino, nitro, cyano, carboxyl, oxo, -C(O)-C₁₋₆ alkyl, C₁₋₆ alkyl, -C(O)-C₁₋₆ alkoxy and C₁₋₆ alkoxy. The base is selected from a purine base, a pyrimidine base, indole, 5-nitroindole or 3-nitropyrrole. The base is selected from purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

### Detailed Description of Embodiments

The disclosure will be further described below in conjunction with examples, but these examples are not meant to limit the scope of the disclosure.

Experimental methods in the embodiments of the disclosure, which do not indicate the specific conditions, are generally in accordance with conventional conditions, or in accordance with the conditions recommended by the raw materials or commodity manufacturer. The reagents for which the specific source is not indicated, are conventional commercially available reagents.

The structures of the compounds are determined by nuclear magnetic resonance (NMR) or /and mass spectrometry (MS). The NMR shift (δ) is given in the unit of 10⁻⁶ (ppm). NMR is determined with a nuclear magnetic resonance spectrometer Bruker AVANCE-400. The solvents for determination are deuterated dimethyl sulfoxide (DMSO-*d₆*), deuterated chloroform (CDCl₃), and deuterated methanol (Methanol-*d₄*), and the internal standard is tetramethylsilane (TMS).

HPLC is determined with Agilent1100 high pressure liquid chromatograph, GAS15B DAD ultraviolet detector and Water Vbridge C18 150*4.6 mm 5 um chromatographic column.

MS is determined with Agilent6120 triple quadrupole mass spectrometer, G1315D DAD detector and Waters Xbridge C18 4.6*50 mm, 5 um chromatographic column. The sample is scanned in a positive/negative ion mode with a mass scan range of 80-1200.

Yantai Huanghai HSGF254 silica gel plate is used as a thin layer chromatography silica plate, and the silica gel plate for the thin layer chromatography (TLC) is of the specification of 0.2 mm ± 0.03 mm, and the specification when separating and purifying a product by thin layer chromatography is 0.4 mm - 0.5 mm.

Combiflash Rf150 (TELEDYNE ISCO) or Isolara one (Biotage) is used for the flash column purification system.

For the normal phase chromatography, Yantai Huanghai silica gel of 200-300 mesh or 300-400 mesh silica gel is generally used as the carrier, or Santai Technologies pre-packed ultra-pure normal phase silica gel column (40-63 µm, 60 g, 24 g, 40 g, 120 g or other specifications) is used.

Known starting materials in the disclosure may be synthesized using or according to methods known in the art, or may be purchased from Shanghai Titan Scientific, ABCR GmbH & Co. KG, Acros Organics, Aldrich Chemical Company, Accela ChemBio Inc., Bide Pharmatech Ltd., and other companies.

If there is no special instruction in the examples, reactions can be carried out under nitrogen atmosphere.

The nitrogen atmosphere means that the reaction bottle is connected to a nitrogen balloon with a volume of about 1 L.

The hydrogen atmosphere means that the reaction bottle is connected to a hydrogen balloon with a volume of about 1 L.

Hydrogen is produced by QPH-1L hydrogen generator from Shanghai Quanpu Scientific Instrument Co., Ltd.

The nitrogen atmosphere or hydrogen atmosphere usually comprises: evacuating, filling with nitrogen or hydrogen, and repeating the operation three times.

Unless otherwise specified in the examples, a solution refers to an aqueous solution.

Unless otherwise indicated, the reaction temperature in the examples refers to room temperature, which is 20°C to 30°C.

The reaction progress in the examples is monitored by thin layer chromatography (TLC). The developing solvent used in the reaction, the eluent system for column chromatography and the developing solvent system for thin layer chromatography used to purify the compound, and the volume ratio of the solvent are adjusted according to the polarity of the compound, and a small amount of alkaline or acidic reagents such as triethylamine and acetic acid can be added for adjustment.

### Example 1 Preparation of (S)-4-benzyl-3-(3-(benzyloxy)propionyl)oxazolidin-2-one (compound 1a)

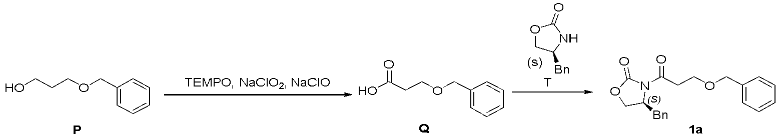

### Step 1) Preparation of compound Q

**Compound P** (20.0 g, 120.3 mmol, 1.0 eq, prepared by the known method "Tetrahedron Letters, 2009, vol. 50, p. 4117 - 4120") was added to a 1 L three-mouth flask, ACN (200 mL), water (200 mL), TEMPO (1.9 g, 12.0 mmol, 0.1 eq) and NaH₂PO₄ dihydrate (93.8 g, 600.0 mmol, 5.0 eq) were added, and the system was stirred for dissolution. NaClO₂ (27.2 g, 300.7 mmol, 2.5 eq, dissolved in 70 mL of water) and 7.5% NaClO aqueous solution (about 12 mL) were successively added; and the obtained system was warmed to 35°C and reacted for 3 hours. After the reaction was complete as monitored in the in-process control, the system was cooled in an ice-water bath, quenched with a solution of sodium sulphite (about 30.0 g, 2.0 eq) in water (150 mL), adjusted to pH = 3 with concentrated HCl, and concentrated under reduced pressure. The aqueous phase was extracted twice with dichloromethane, the organic phase was dried over anhydrous sodium sulphate, filtered and concentrated under reduced pressure to obtain a crude (about 22.3 g). The crude was slurried with 200 mL of (PE : EA=10 : 1) for 10 min and filtered, and the filtrate was concentrated and dried with suction to obtain a product (21.0 g, yield: 97%).

MS-ESI: m/z 203.1 [M+Na]⁺

### Step 2) Preparation of compound 1a

**Compound Q** (10.0 g, 55.6 mmol, 1.0 eq) was added to a 500 mL three-mouth flask, anhydrous THF (150 mL) was added, and the system was evacuated and placed under nitrogen protection, and cooled to an internal temperature of - 20°C. Triethylamine (11.3 g, 111.2 mmol, 2.0 eq) was added, and pivaloyl chloride (8.0 g, 66.7 mmol, 1.2 eq) was added dropwise at -20°C. To the obtained system was added anhydrous lithium chloride (2.8 g, 66.7 mmol, 1.2 eq), and then the dropwise addition of **compound T** (9.9 g, 55.6 mmol, 1.0 eq in 50 mL THF solution) was started. After the dropwise addition was completed, the system was naturally warmed to room temperature and reacted for 3 h. After the reaction was basically complete as monitored in the in-process control, the system was cooled in an ice-water bath, 1 N HCl aqueous solution (150 mL) was added, and the obtained system was stirred for 10 min and concentrated under reduced pressure. The aqueous phase was extracted three times with EA, and the EA phases were combined, washed twice with saturated NaHCO₃ aqueous solution, dried over anhydrous sodium sulphate, filtered and concentrated under reduced pressure to obtain a crude (about 19 g). To the crude (19 g) was added isopropanol (95 mL), and the system was heated to 60°C for complete dissolution. The heating was stopped, the system was naturally cooled to 20°C, slurried for 16 hours and filtered, and the filter cake was washed twice with isopropanol (8 mL × 2) and dried with suction to obtain a product (11.8 g, yield: 62%).

MS-ESI: m/z 362.1 [M+Na]⁺

### Example 2 Preparation of (S)-2-amino-9-(3-(benzyloxy)-2-(hydroxymethyl)propyl)-1,9-dihydro-6H-purin-6-one (compound 1e) (route 1)

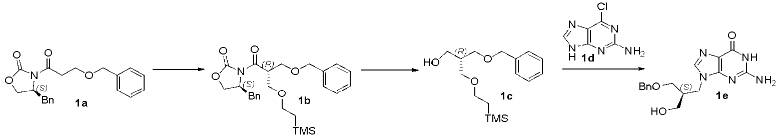

### Step 1) Preparation of compound 1b

**Compound 1a** (7.1 g, 20.94 mmol, 1.0 eq) was added to a 250 mL three-mouth flask, NaI (6.3 g, 41.9 mmol, 2.0 eq) was added, and the system was evacuated and placed under nitrogen protection. Anhydrous THF (140 mL) was added, the obtained system was stirred and cooled to an internal temperature of - 60°C, SEMCl (5.3 g, 1.5 eq) was added, and then the dropwise addition of LiHMDS (1 M, 27.2 mL, 1.3 eq) was started. After the dropwise addition was completed, the system was reacted at -60°C for 30 min to 1 h. After the reaction was complete as monitored in the in-process control, the system was warmed to - 40°C to -20°C and reacted for 30 min to 1 h. The reaction was quenched with saturated sodium chloride aqueous solution (100 mL) and diluted in MTBE (100 mL) for liquid separation. The aqueous phase was extracted again with MTBE (100 mL), and the organic phases were combined, washed once with saturated sodium chloride (100 mL), dried over anhydrous sodium sulphate, filtered and concentrated under reduced pressure to obtain a crude (about 21 g), which was purified by column chromatography to obtain **compound 1b** (4.92 g, yield: 50%).
MS-ESI: m/z 492.4 [M+Na]⁺
¹H NMR (CDCl₃, 400M): 0.01 (s, 9H), 0.89~0.93 (m, 2H), 2.76∼2.82 (m, 1H), 3.23∼3.28 (m, 1H), 3.51∼3.55 (m, 2H), 3.67∼3.72 (m, 2H), 3.75∼3.79 (m, 1H), 3.83∼3.88 (m, 1H),4.12~4.14 (m, 2H), 4.38~4.41 (m, 1H), 4.49~4.57 (m, 2H),4.68∼4.72 (m, 1H), 7.22∼7.35 (m, 10H).

### Step 2) Preparation of compound 1c

**Compound 1b** (2.0 g, 4.25 mmol, 1.0 eq) was added to a 250 mL flask, THF (30 mL) and water (10 mL) were added, and the system was stirred for dissolution. In an ice-water bath, NaBH₄ (485 mg, 3.0 eq) was added in batches. The ice-water bath was removed, and the system was reacted at room temperature for 16 hours. After the reaction was complete as monitored in the in-process control, to the system was added water (10 mL), and the obtained system was concentrated under reduced pressure. The aqueous phase was extracted three times with MTBE, and the organic phases were combined, dried over anhydrous sodium sulphate, filtered and concentrated under reduced pressure to obtain a crude (about 2 g). The crude was slurried with n-heptane (20 mL) for 30 min and filtered, the filter cake was washed twice with n-heptane, and the filtrate was collected, diluted in MTBE (10 mL), washed twice with water, dried over anhydrous sodium sulphate, filtered and concentrated to obtain **compound 1c** (1.08 g, yield: 87%).
ESI: m/z 319.3 [M+Na]⁺
¹H NMR (CDCl₃, 400M): 0.01 (s, 9H), 0.89~0.93 (m,3H), 3.46~3.59 (m, 6H), 3.77 (t, 2H), 4.50 (s, 2H), 7.25∼7.34 (m, 5H).

### Step 3) Preparation of compound 1e

**Compound 1d** (1.12 g, 6.58 mmol, 1.0 eq) was added to a 250 mL flask, PPh₃ (3.46 g, 13.16 mmol, 2.0 eq) was added, and the system was evacuated and placed under nitrogen protection. **Compound 1c** (1.95 g, 6.58 mmol, 1.0 eq) was dissolved in anhydrous THF (50 mL) and added to the system. The obtained system was placed in an oil bath and heated to an internal temperature of 60°C, and the dropwise addition of DIAD (2.66 g, 13.16 mmol, 2.0 eq) was started. After the dropwise addition was completed, the system was reacted at 60°C for 10 min. After the reaction was complete as monitored in the in-process control, the system was cooled to room temperature, 6 N HCl aqueous solution (12.5 mL) was added, and the obtained system was heated to an external temperature of 80°C and reacted for 24 hours. After the reaction was complete as monitored in the in-process control, the system was cooled to room temperature, water (10 mL) and MTBE (30 mL) were added, and the obtained system was stirred for liquid separation. The aqueous phase was then back-extracted twice with dichloromethane and adjusted to a basic pH with sodium carbonate solid to precipitate a solid, the system was filtered, and the filter cake was washed with water (10 mL) and dried to obtain **compound 1e** (1.9 g, yield: 87%).
ESI: m/z 330.3 [M+H]⁺
¹H NMR ((CD₃)₂SO, 400M): 2.20~2.29 (m, 1H), 3.30∼3.72 (m, 4H),3.90∼4.05 (m,2H), 4.30∼4.50 (m, 2H), 4.68∼4.72 (m, 1H), 6.49 (br s, 2H), 7.31~7.63 (m, 6H), 10.58 (br s, 1H).

### Example 3 Preparation of (S)-2-amino-9-(3-(benzyloxy)-2-(hydroxymethyl)propyl)-1,9-dihydro-6H-purin-6-one (compound 1e) (route 2)

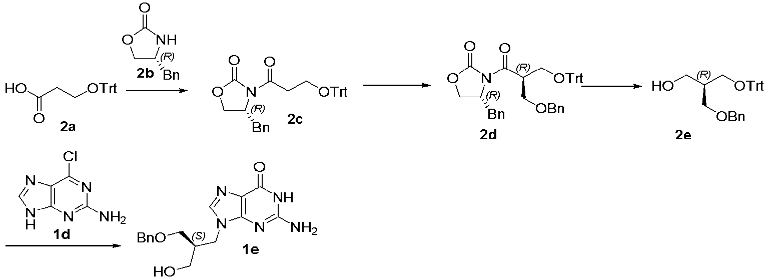

### Step 1) Preparation of compound 2c

**Compound 2a** (18.5 g, 55.6 mmol, 1.0 eq, prepared by the known method "Journal of Medicinal Chemistry, 2004, vol. 47, p. 1487 - 1513") was added to a 500 mL three-mouth flask, anhydrous THF (150 mL) was added, and the system was evacuated and placed under nitrogen protection, and cooled to an internal temperature of -20°C. Triethylamine (11.3 g, 111.2 mmol, 2.0 eq) was added, and pivaloyl chloride (8.0 g, 66.7 mmol, 1.2 eq) was added dropwise. After the dropwise addition was completed, the system was reacted at an internal temperature of -20°C for 45 min. To the system was added anhydrous lithium chloride (2.8 g, 66.7 mmol, 1.2 eq), and then the dropwise addition of **compound 2b** (9.9 g, 55.6 mmol, 1.0 eq in 50 mL THF solution) was started. After the dropwise addition was completed, the system was naturally warmed to room temperature and reacted for 3 h. After the reaction was complete as monitored in the in-process control, the system was cooled in an ice-water bath, 1 N HCl aqueous solution (150 mL) was added, and the obtained system was stirred for 10 min and concentrated under reduced pressure to remove THF. The aqueous phase was extracted three times with EA, and the EA phases were combined and washed twice with saturated NaHCO₃ aqueous solution, dried over anhydrous sodium sulphate, filtered and concentrated under reduced pressure to obtain a crude (about 34 g). Isopropanol (95 mL) was added, and the system was heated to 60°C for complete dissolution. The heating was stopped, the system was naturally cooled to 20°C, slurried for 16 hours and filtered, and the filter cake was washed twice with isopropanol (8 mL) and dried with suction to obtain **compound 2c** (16.4 g, yield: 60%).
ESI: m/z 514.4 [M+Na]⁺
¹H NMR (400 M,CDCl₃) *δ* (ppm) 2.71~2.77 (m, 1H), 3.26~3.72 (m, 3H), 3.47∼3.51 (m, 2H), 4.14∼4.16 (m, 2H), 4.64∼4.69 (m, 1H), 7.19~7.34 (m, 14H), 7.42~7.46 (m, 6H).

### Step 2) Preparation of compound 2d

**Compound 2c** (2.951 g, 6.0 mmol, 1.0 eq) was added to a 250 mL three-mouth flask, NaI (2.702 g, 18 mmol, 3.0 eq) was added, and the system was evacuated and placed under nitrogen protection. Anhydrous THF (60 mL) was added, the obtained system was stirred and cooled to an internal temperature of - 60°C, and BOMCl (1.13 g, 1.2 eq) was added. The dropwise addition of LiHMDS (1 M, 6 mL, 1.0 eq) was started. After the dropwise addition was completed, the system was reacted at -60°C for 30 min, BOMCl (1.04 g, 1.1 eq) was added, and LiHMDS (1 M, 6 mL, 1.0 eq) was added dropwise. After the dropwise addition was completed, the system was reacted at -60°C for 30 min. After the reaction was complete as monitored in the in-process control, the system was warmed to -40°C to -20°C and reacted for 30 min. The reaction was quenched with saturated sodium chloride aqueous solution and diluted in water (20 mL) and MTBE (50 mL) for liquid separation. The aqueous phase was extracted again with MTBE (50 mL), and the organic phases were combined, washed once with saturated sodium chloride (25 mL), dried over anhydrous sodium sulphate, filtered and concentrated under reduced pressure to obtain a crude (6.19 g), which was separated by column chromatography to obtain **compound 2d** (2.17 g, yield: 59%).
MS m/z (ESI): 634.4 [M+Na]⁺
¹H NMR (400 MHz, CDCl₃): δ 7.39~7.18 (m, 25 H), 4.69~4.63 (m, 1 H), 4.55∼4.50 (m, 3 H), 4.15∼4.08 (m, 2 H), 3.91 (dd, 1 H), 3.76 (dd, 1 H),3.42 (d, 2 H), 3.24 (dd, 1 H),2.77 (dd, 1 H).

### Step 3) Preparation of compound 2e

**Compound 2d** (2.134 g, 3.19 mmol, 1.0 eq) was added to a reaction flask, THF (24 mL) and water (8 mL) were added, and the system was stirred for dissolution. In an ice-water bath, NaBH₄ (403 mg, 3.0 eq) was added in batches. The ice-water bath was removed, and the system was reacted at room temperature for 23 hours. After the reaction was complete as monitored in the in-process control, to the system was added water (20 mL), the aqueous phase was extracted three times with MTBE, and the organic phases were combined, washed once with water, dried over anhydrous sodium sulphate, filtered and concentrated under reduced pressure to obtain a crude (about 2.22 g). To the crude were added n-heptane (45 mL) and MTBE (9 mL), and the system was heated to 60°C, slurried, stirred for 1 h, naturally cooled to room temperature and filtered. The filter cake was washed with a mixture of n-heptane/MTBE = 5 : 1 (9 mL), and the filtrate was collected, diluted in MTBE (10 mL), washed twice with water, dried over anhydrous sodium sulphate, filtered and concentrated to obtain an oil (1.608 g, yield: 105%).
MS m/z (ESI): 461.4 [M+Na]⁺
¹H NMR (400 MHz, CDCl₃): δ 7.42~7.19 (m, 20 H), 4.49 (s, 2 H), 3.76 (t, 2 H), 3.70∼3.60 (m, 2 H), 3.25 (d, 2 H), 2.47 (t, 1 H), 2.22∼2.15 (m, 1 H).

### Step 4) Preparation of compound 1e

**Compound 1d** (492 mg, 2.90 mmol, 1.0 eq) was added to a 250 mL flask, PPh₃ (1.51 g, 5.76 mmol, 2.0 eq) was added, and the system was evacuated and placed under nitrogen protection. **Compound 2e** (1.34 g, 3.06 mmol, 1.05 eq) was dissolved in anhydrous THF (26 mL) and added to the system. DIAD (1.17 g, 5.79 mmol, 2.0 eq) was added dropwise, and after the dropwise addition was completed, the obtained system was reacted for 24 h. After the reaction was complete as monitored in the in-process control, 6 N HCl aqueous solution (13 mL) was added, and the system was heated to 60°C and reacted for 10 hours. After the reaction was complete as monitored in the in-process control by LCMS, the system was cooled to room temperature, water (10 mL) and MTBE (20 mL) were added, and the obtained system was stirred for liquid separation. The aqueous phase was then back-extracted twice with dichloromethane and adjusted to a basic pH with sodium carbonate solid to precipitate a solid, the system was filtered, and the filter cake was washed with water (10 mL) and dried to obtain **compound 1e** (597 mg, yield: 62%).
MS m/z (ESI): 330.2[M+H]⁺.

### Example 4 Preparation of (R)-N-(9-(3-(bis(4-methoxyphenyl)(phenyl)methoxy)-2-(((tert-butyldimethylsilyl)oxy)methyl)propyl)-6-oxo-6,9-dihydro-1H-purin-2-yl)isobutyramide (compound 1h)

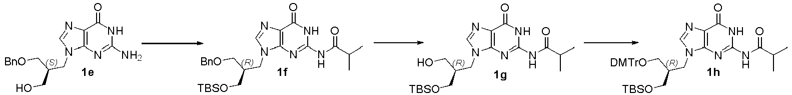

### Step 1) Preparation of compound 1f

**Compound 1e** (1.80 g, 5.47 mmol, 1.0 eq) was added to a 50 mL flask, anhydrous pyridine (20 mL) was added, and the system was stirred for dissolution, evacuated and placed under nitrogen protection. TBSCl (1.65 g, 10.94 mmol, 2.0 eq) was added, and the obtained system was reacted at 25°C for 16 hours. After the reaction was complete as monitored in the in-process control, the system was cooled in an ice-water bath, iPrCOCl (0.61 g, 6.02 mmol, 1.1 eq) was added, and the obtained system was reacted at 0-25°C for 4 hours. After the reaction was complete as monitored in the in-process control, the following post-treatment was performed: the system was quenched with water (30 mL) and extracted three times with MTBE, and the organic phases were combined, dried over anhydrous sodium sulphate, filtered and concentrated to obtain a crude, which was purified by column chromatography (elution solvent: EA) to obtain **compound 1f** (1.4 g, yield: 50%).
ESI: m/z 514.4 [M+H]⁺
¹H NMR (CDCl₃, 400M): 0.00 (s, 6H),0.91 (s,9H), 1.23~1.25 (d, 6H), 2.33∼2.36 (m, 1H), 2.47∼2.51 (m, 1H), 3.38∼3.45 (m, 2H), 3.53∼3.63 (m, 2H),4.15 (d, 2H), 4.68 (s, 2H) ,7.31~7.39 (m, 5H), 7.59 (s, 1H), 7.96 (s, 1H), 11.87 (s, 1H).

### Step 2) Preparation of compound 1g

**Compound 1f** (450 mg, 0.876 mmol, 1.0 eq) was added to a 100 mL singleneck flask, isopropanol/water (5v/1v, 9 mL) and Pd(OH)₂/C (225 mg, 20% Pd, 50%wt) were added, and the system was subjected to gas replacement by a hydrogen balloon, heated to 60°C and reacted for 24 hours. After the reaction was complete as monitored in the in-process control, the reaction system was cooled to room temperature and filtered, the filter cake was rinsed twice with dichloromethane, and the filtrate was subjected to rotary evaporation to dryness to obtain **compound 1g** (371 mg, yield: 90%).
ESI: m/z 424.4 [M+H]⁺
¹H NMR (CDCl₃, 400 M) *δ* (ppm) 0.05 (s, 6H), 0.91 (s, 9H), 1.25 (d, 6H), 2.12-2.23 (m, 1H), 2.73-2.85 (m, 1H), 3.40-3.50 (m, 2H), 3.51-3.63 (m, 2H), 4.24 (d, 2H), 7.66 (s, 1H).

### Step 3) Preparation of compound 1h

**Compound 1g** (100 mg, 0.236 mmol, 1.0 eq) was added to a 25 mL singleneck flask, pyridine (5 mL) was added, and the system was stirred for dissolution under nitrogen protection. DMTrCl (200 mg, 0.59 mmol) was added, and the obtained system was reacted at room temperature for 3 hours. After the reaction was complete as monitored in the in-process control, to the system were added MTBE (20 mL) and water (10 mL), the obtained system was stirred, and the layers were separated. The organic phase was washed with saturated sodium chloride aqueous solution, dried over anhydrous magnesium sulphate, filtered and subjected to rotary evaporation to dryness to obtain a crude (300 mg).
ESI: m/z 726.5 [M+H]⁺

### Example 5 Preparation of (R)-N-(9-(3-(bis(4-methoxyphenyl)(phenyl)methoxy)-2-(((tert-butyldiphenylsilyl)oxy)methyl)propyl)-6-oxo-6,9-dihydro-1H-purin-2-yl)isobutyramide (compound 2h)

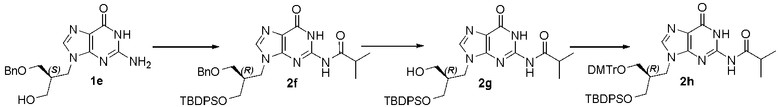

### Step 1) Preparation of compound 2f

**Compound 1e** (200 mg, 0.61 mmol, 1.0 eq) was added to a 25 mL flask, anhydrous pyridine (3 mL) was added, and the system was stirred for dissolution, evacuated and placed under nitrogen protection. TBDPSCl (345 g, 1.26 mmol, 2.0 eq) was added, and the obtained system was reacted for 16 hours. After the reaction was complete as monitored in the in-process control, the system was cooled in an ice-water bath, iPrCOCl (68 mg, 0.67 mmol, 1.1 eq) was added, and the obtained system was reacted for 4 hours. After the reaction was complete as monitored in the in-process control, the system was quenched with water (10 mL) and extracted with MTBE (10 mL × 3), and the organic phases were combined, dried over anhydrous sodium sulphate, filtered and concentrated to obtain a crude, which was purified by column chromatography to obtain **compound 2f** (180 mg, yield: 46%).
ESI: m/z 638.5 [M+H]⁺
¹H NMR (CDCl₃, 400M): 1.07 (s, 9H), 1.19~1.21 (m, 6H), 1.16∼1.29 (m, 1H),2.34∼2.43 (m, 1H), 3.40∼3.46 (m, 2H), 3.66∼3.69 (m, 2H),4.18~4.22 (m, 2H), 4.43 (s, 2H), 7.26~7.83 (m, 17H), 11.86 (s, 1H).

### Step 2) Preparation of compound 2g

**Compound 2f** (500 mg, 0.78 mmol, 1.0 eq) was added to a 25 mL flask, anhydrous dichloromethane (10 mL) was added, and the system was stirred for dissolution, evacuated and placed under nitrogen protection, and cooled to -65°C. A solution of BCl₃ in toluene (1.0 M/L, 3.9 mL, 3.9 mmol, 5.0 eq) was slowly added dropwise, and after the dropwise addition was completed, the obtained system was reacted at -60°C to 65°C for 30 min. After the reaction was complete as monitored in the in-process control, the reaction was quenched with a solution of ammonia in methanol (7 mol/L, 4 mL) and warmed to 25°C. The system was reacted for 2 hours, water (20 mL) and dichloromethane (20 mL) were added, and the obtained system was stirred for liquid separation. The organic phase was collected, the aqueous phase was extracted twice with dichloromethane, and the organic phases were combined, washed twice with water, dried over anhydrous sodium sulphate, filtered, and concentrated under pressure to obtain a crude, which was purified by column chromatography to obtain compound 2g (280 mg, yield: 65%).
ESI: m/z 548.4 [M+H]⁺

### Step 3) Preparation of compound 2h

**Compound 2g** (224 mg, 0.41 mmol, 1.0 eq) was added to a 25 mL singleneck flask, pyridine (2.3 mL) was added, and the system was stirred for dissolution under nitrogen protection. DMTrCl (278 mg, 0.82 mmol) was added, and the obtained system was reacted at room temperature for 3 hours. After the reaction was complete as monitored in the in-process control, to the system were added MTBE (20 mL) and water (10 mL), the obtained system was stirred, and the layers were separated. The organic phase was washed with saturated sodium chloride aqueous solution, dried over anhydrous magnesium sulphate, filtered and subjected to rotary evaporation to dryness to obtain a crude, which was purified by column chromatography to obtain an oil product (310 mg, yield: 89%).
MS m/z (ESI): 850.6[M+H]⁺
¹H NMR (400 MHz, CDCl₃): δ 11.81 (s, 1 H) , 7.65~7.20 (m, 21 H), 6.78∼6.75 (m, 4 H), 4.18~4.10 (m, 2 H), 3.78∼3.66 (m, 8 H), 3.20~3.08 (m, 2 H), 2.47∼2.44 (m, 1 H), 2.24-2.2.21 (m, 1 H),1.22 (s, 3 H), 1.20 (s, 3 H), 1.02 (s, 9 H).

### Example 6 Preparation of (S)-N-(9-(3-(bis(4-methoxyphenyl)(phenyl)methoxy)-2-(hydroxymethyl)propyl)-6-oxo-6,9-dihydro-1H-purin-2-yl)isobutyramide (compound 1i) (route 1)

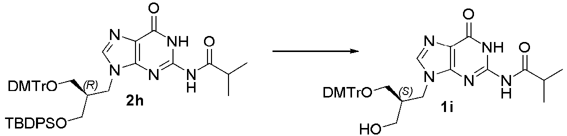

**Compound 2h** (310 mg, 0.36 mmol, 1.0 eq) was added to a 25 mL singleneck flask, TBAF (2 mL, 1.0 M in THF, 2 mmol) was added, and the system was stirred at room temperature for dissolution and reacted for 1 hour. After the reaction was complete as monitored in the in-process control, to the system were added ethyl acetate (20 mL) and water (10 mL), the obtained system was stirred, and the layers were separated. The organic phase was washed with saturated sodium chloride aqueous solution, dried over anhydrous magnesium sulphate, filtered and subjected to rotary evaporation to dryness to obtain a crude (310 mg). The crude was subjected to ACN/H₂O and petroleum ether/MTBE systems for slurrying and purifying to obtain the pure product, **compound 1i** (200 mg, yield: 91%).
ESI: m/z 612.4 [M+H]⁺
¹H NMR (400 M, (CD₃)₂SO) δ (ppm), 12.03 (s, 1H),11.58 (s, 1H), 7.81 (s, 1H), 7.23-7.26 (m, 4H), 7.18-7.20 (m, 1H), 7.10-7.13 (m, 4H), 6.79-6.82 (m,4H), 4.72-4.74 (m, 1H), 4.05-4.10 (m, 2H), 3.72-3.74 (m, 6H),3.49-3.52 (m, 2H), 3.02-3.06 (m, 1H),2.87-2.91 (m, 1H), 2.77-2.80 (m, 1H), 2.33-2.36 (m, 1H), 1.11-1.13 (m, 6H).

### Example 7 Preparation of (S)-N-(9-(3-(bis(4-methoxyphenyl)(phenyl)methoxy)-2-(hydroxymethyl)propyl)-6-oxo-6,9-dihydro-1H-purin-2-yl)isobutyramide (compound 1i) (route 2)

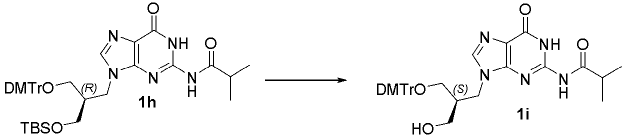

**Compound 1h** (300 mg, crude, 0.236 mmol) was added to a 25 mL singleneck flask, TBAF (2 mL, 1.0 M in THF, 2 mmol) was added, and the system was stirred at room temperature for dissolution and reacted for 1 hour. After the reaction was complete as monitored in the in-process control, to the system were added ethyl acetate (20 mL) and water (10 mL), the obtained system was stirred, and the layers were separated. The organic phase was washed with saturated sodium chloride aqueous solution, dried over anhydrous magnesium sulphate, filtered and subjected to rotary evaporation to dryness to obtain a crude (250 mg). The crude was subjected to ACN/H₂O and petroleum ether/MTBE systems for slurrying and purifying to obtain **compound 1i** (110 mg, two-step yield: 76%).
ESI: m/z 612.4 [M+H]⁺
¹H NMR (400 M, (CD₃)₂SO) δ (ppm) 12.03 (s, 1H),11.58 (s, 1H), 7.81 (s, 1H), 7.23-7.26 (m, 4H), 7.18-7.20 (m, 1H), 7.10-7.13 (m, 4H), 6.79-6.82 (m,4H), 4.72-4.74 (m, 1H), 4.05-4.10 (m, 2H), 3.72-3.74 (m, 6H),3.49-3.52 (m, 2H), 3.02-3.06 (m, 1H),2.87-2.91 (m, 1H), 2.77-2.80 (m, 1H), 2.33-2.36 (m, 1H), 1.11-1.13 (m, 6H).

### Example 8 Preparation of (R)-3-(bis(4-methoxyphenyl)(phenyl)methoxy)-2-((2-isobutyrylamino-6-oxo-1,6-dihydro-9H-purin-9-yl)methyl)propyl(2-cyanoethyl)diisopropylphosphoramidite (compound 1)

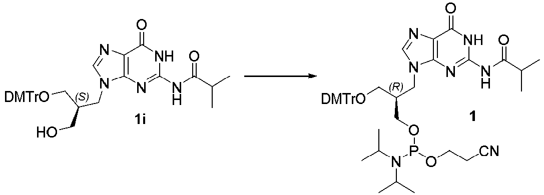

Under nitrogen protection, to a pre-dried reaction flask R1 were added **compound 1i** (3.0 g, 4.91 mmol, 1.0 eq), tetrazole (172 mg, 2.46 mmol, 0.5 eq), 1-methylimidazole (81 mg, 0.99 mmol, 0.2 eq) and powdered 3A molecular sieve (0.45 g, 15%wt), the system was subjected to nitrogen replacement three times and anhydrous acetonitrile (30 mL) was added. The obtained system was stirred at room temperature for 10 min for dissolution, bis(diisopropylamino)(2-cyanoethoxy)phosphine (1.92 g, 6.37 mmol, 1.3 eq) was added, and the system was stirred at room temperature for 2 h. After the reaction was complete as monitored in the in-process control, the reaction solution was filtered to remove the molecular sieve, and then the filtrate was added to a reaction flask R2 containing water (300 mL). The system was stirred at room temperature for 30 min to 1 h, and a viscous solid gradually precipitated. The obtained system was filtered to remove the supernatant, the solid was washed with water, dissolved in MTBE (240 mL), washed with saturated sodium bicarbonate aqueous solution, DMF/water (1 : 1), water and saturated NaCl, dried over anhydrous sodium sulphate, filtered and concentrated under reduced pressure to obtain a crude (3.922 g). The crude was purified by filtration on alumina and silica gel, the eluate was collected and concentrated under reduced pressure, and the residue was dried using an oil pump to obtain a product (2.84 g, yield: 71.2%).
ESI: m/z 812.2 [M+H]⁺
¹H NMR (400 M,CDCl₃) *δ* (ppm) 11.9 (br s, 1H), 8.70-9.01 (m, 1H), 7.19-7.43 (m, 10H), 6.78-6.83 (m,4H), 4.25-4.28 (m, 1H), 4.11-4.16 (m, 1H), 3.69-3.86 (m, 9H),3.48-3.57 (m, 3H), 3.03-3.24 (m, 2H),3.58-3.67 (m, 3H), 2.41-2.43 (m, 1H), 1.03-1.29 (m, 18H).

## Claims

1. A method for preparing a compound of formula VIII or a salt thereof, 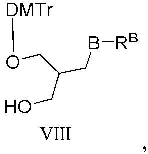 **characterized in that** the method comprises the steps of:
a) converting a compound of formula II into a compound of formula III,
b) converting the compound of formula III into a compound of formula IV,
c) converting the compound of formula IV into a compound of formula V,
d) converting the compound of formula V into a compound of formula VI,
e) reacting the compound of formula VI with 4,4'-dimethoxytrityl halide to form a compound of formula VII, and
f) converting the compound of formula VII into the compound of formula VIII, 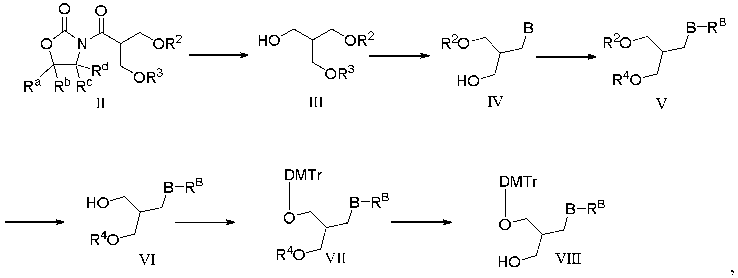
wherein
R^{a}, R^{b} and R^{c} are the same or different, and are each independently selected from hydrogen, halogen, hydroxyl, amino, C₁₋₆ alkyl, C₁₋₆ alkoxy, 3- to 10-membered cycloalkyl, 3- to 10-membered heterocyclyl, 6- to 10-membered aryl or 5- to 10-membered heteroaryl, wherein the alkyl, alkoxy, cycloalkyl, heterocyclyl, aryl and heteroaryl are optionally substituted with one or more substituents selected from halogen, hydroxyl, amino, C₁₋₆ alkyl or C₁₋₆ alkoxy;
R^{d} is selected from benzyl, phenyl, biphenyl or C₁₋₆ alkyl, wherein the benzyl, phenyl, biphenyl and C₁₋₆ alkyl are optionally substituted with one or more substituents selected from halogen, hydroxyl, amino, C₁₋₆ alkyl or C₁₋₆ alkoxy;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl;
R² and R⁴ are the same or different, and are each independently a hydroxyl protecting group;
R³ is selected from trityl, tert-butyldimethylsilyl, tert-butyldiphenylsilyl, (trimethylsilyl)ethyl or 2-tetrahydropyran.

2. The method according to claim 1, **characterized in that** R^{a}, R^{b} and R^{c} are all hydrogen.

3. The method according to claim 1 or 2, **characterized in that** R^{d} is benzyl.

4. The method according to any one of claims 1 to 3, **characterized in that** R² is selected from benzyl, trityl, tert-butyldimethylsilyl, tert-butyldiphenylsilyl, (trimethylsilyl)ethyl or 2-tetrahydropyran, preferably benzyl.

5. The method according to any one of claims 1 to 4, **characterized in that** the compound of formula VIII is a compound of formula VIII-1, 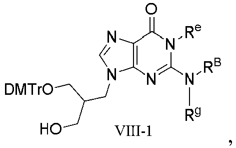 and the method comprises the steps of converting a compound of formula II-1 into a compound of formula III-1, and reacting the compound of formula III-1 with a compound of formula D to form a compound of formula IV-1, 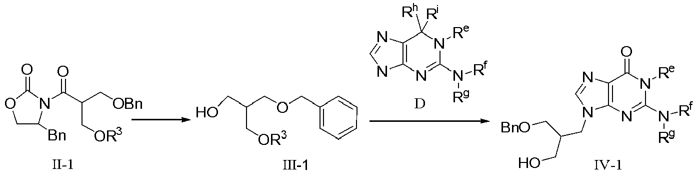
wherein R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl;
R^{f}, R^{h} and Rⁱ are the same or different, and are each independently selected from hydrogen, halogen, hydroxyl, amino, C₁₋₆ haloalkyl, C₁₋₆ hydroxy-substituted alkyl or C₁₋₆ amino-substituted alkyl;
alternatively, R^{h} and Rⁱ together with the carbon atom to which they are attached form an oxo group or a thio group;
R³, R⁴ and R^{B} are as defined in claim 1.

6. The method according to claim 5, **characterized in that** the method further comprises the steps of reacting the compound of formula IV-1 with an acyl halide compound X¹-R^{B} to form a compound of formula V-1, and converting the compound of formula V-1 into a compound of formula VI-1, 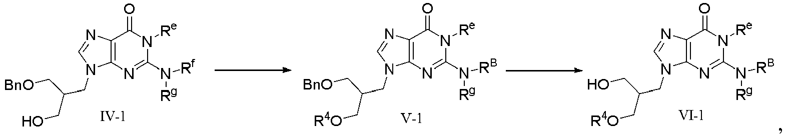
wherein in the acyl halide compound, X¹ is connected to the carbonyl on R^{B}; X¹ represents halogen and is selected from fluorine, chlorine, bromine or iodine, preferably chlorine;
R⁴ and R^{B} are as defined in claim 1, and R^{e}, R^{f} and R^{g} are as defined in claim 5.

7. The method according to claim 5 or 6, **characterized in that** the method further comprises the steps of reacting the compound of formula VI-1 with DMTr-X² to form a compound of formula VII-1, and converting the compound of formula VII-1 into the compound of formula VIII-1, 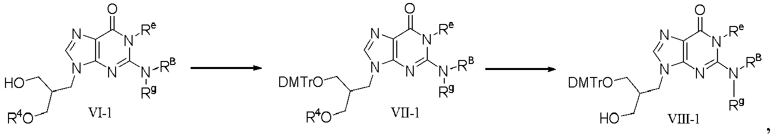
wherein X² represents halogen and is selected from fluorine, chlorine, bromine or iodine, preferably chlorine;
R⁴ and R^{B} are as defined in claim 1, and R^{e}, R^{f} and R^{g} are as defined in claim 5.

8. The method according to any one of claims 5 to 7, **characterized in that** the method comprises the steps of:
a) converting the compound of formula II-1 into the compound of formula III-1,
b) reacting the compound of formula III-1 with the compound of formula D to form the compound of formula IV-1,
c) reacting the compound of formula IV-1 with the acyl halide compound X¹-R^{B} to form the compound of formula V-1,
d) converting the compound of formula V-1 into the compound of formula VI-1,
e) reacting the compound of formula VI-1 with DMTr-X² to form the compound of formula VII-1, and
f) converting the compound of formula VII-1 into the compound of formula VIII-1 , 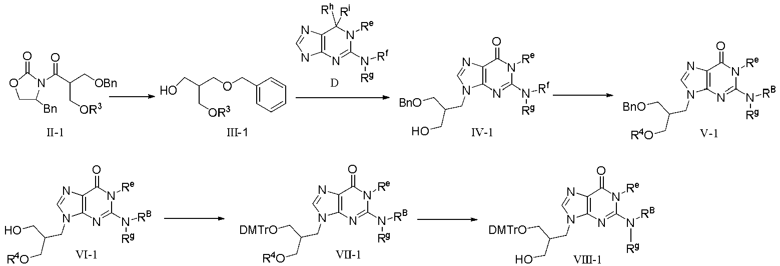 wherein R³, R⁴ and R^{B} are as defined in claim 1, R^{e}, R^{f}, R^{g}, R^{h} and Rⁱ are as defined in claim 5, X¹ is as defined in claim 6, and X² is as defined in claim 7.

9. The method according to any one of claims 1 to 8, **characterized in that** B is selected from substituted or unsubstituted purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole, preferably substituted or unsubstituted adenine, guanine, cytosine, uracil or thymine, and most preferably substituted or unsubstituted guanine.

10. The method according to any one of claims 1 to 9, **characterized in that** R³ is selected from trimethylsilyl or trityl.

11. The method according to any one of claims 1 to 10, **characterized in that** R⁴ is selected from trityl, tert-butyldimethylsilyl, tert-butyldiphenylsilyl, (trimethylsilyl)ethyl or 2-tetrahydropyran, preferably tert-butyldimethylsilyl or tert-butyldiphenylsilyl.

12. The method according to any one of claims 1 to 11, **characterized in that** R^{B} is C(O)-C₁₋₆ alkyl, preferably -C(O)-CH₃, -C(O)-CH₂CH₃ or -C(O)-CH(CH₃)₂, and most preferably -C(O)-CH(CH₃)₂.

13. The method according to any one of claims 5 to 12, **characterized in that** R^{h} is hydrogen, and Rⁱ is halogen, wherein the halogen is selected from fluorine, chlorine, bromine or iodine, preferably chlorine.

14. The method according to any one of claims 5 to 13, **characterized in that** R^{e}, R^{f} and R^{g} are all hydrogen.

15. A compound represented by formula II or a pharmaceutically acceptable salt thereof 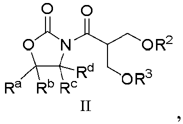 preferably a compound represented by formula II-1 or a pharmaceutically acceptable salt thereof 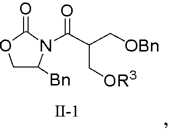 **characterized in that**
R^{a}, R^{b} and R^{c} are the same or different, and are each independently selected from hydrogen, halogen, hydroxyl, amino, C₁₋₆ alkyl, C₁₋₆ alkoxy, 3- to 10-membered cycloalkyl, 3- to 10-membered heterocyclyl, 6- to 10-membered aryl or 5- to 10-membered heteroaryl, wherein the alkyl, alkoxy, cycloalkyl, heterocyclyl, aryl and heteroaryl are optionally substituted with one or more substituents selected from halogen, hydroxyl, amino, C₁₋₆ alkyl or C₁₋₆ alkoxy;
R^{d} is selected from benzyl, phenyl, biphenyl or C₁₋₆ alkyl, wherein the benzyl, phenyl, biphenyl and C₁₋₆ alkyl are optionally substituted with one or more substituents selected from halogen, hydroxyl, amino, C₁₋₆ alkyl or C₁₋₆ alkoxy;
R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl;
R³ is selected from trityl, tert-butyldimethylsilyl, tert-butyldiphenylsilyl, (trimethylsilyl)ethyl or 2-tetrahydropyran, preferably trimethylsilyl or trityl.

16. A compound represented by formula III or a pharmaceutically acceptable salt thereof 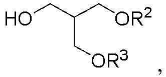 preferably a compound represented by formula III-1 or a pharmaceutically acceptable salt thereof 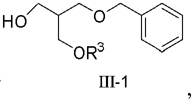
characterized in thatR² is a hydroxyl protecting group, preferably benzyl, trityl, tert-butyldimethylsilyl, tert-butyldiphenylsilyl, (trimethylsilyl)ethyl or 2-tetrahydropyran, and preferably benzyl;
R³ is selected from trityl, tert-butyldimethylsilyl, tert-butyldiphenylsilyl, (trimethylsilyl)ethyl or 2-tetrahydropyran, preferably trimethylsilyl or trityl.

17. A compound represented by formula IV or a pharmaceutically acceptable salt thereof 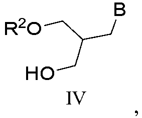 preferably a compound represented by formula IV-1 or a pharmaceutically acceptable salt thereof 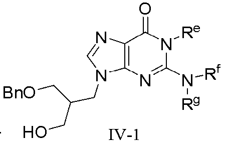
characterized in thatR² is a hydroxyl protecting group, preferably benzyl, trityl, tert-butyldimethylsilyl, tert-butyldiphenylsilyl, (trimethylsilyl)ethyl or 2-tetrahydropyran, and preferably benzyl;
B is a base;
R^{e}, R^{f} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl.

18. A compound represented by formula V or a pharmaceutically acceptable salt thereof 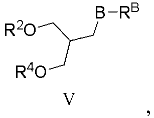 preferably a compound represented by formula V-1 or a pharmaceutically acceptable salt thereof, 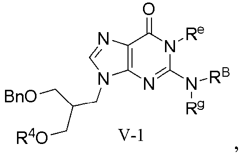 **characterized in that**
R² is a hydroxyl protecting group, preferably benzyl, trityl, tert-butyldimethylsilyl, tert-butyldiphenylsilyl, (trimethylsilyl)ethyl or 2-tetrahydropyran, and most preferably benzyl;
R⁴ is a hydroxyl protecting group, preferably trityl, tert-butyldimethylsilyl, tert-butyldiphenylsilyl, (trimethylsilyl)ethyl or 2-tetrahydropyran, and most preferably tert-butyldimethylsilyl or tert-butyldiphenylsilyl;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl, preferably -C(O)-CH₃, -C(O)-CH₂CH₃ or -C(O)-CH(CH₃)₂, and most preferably -C(O)-CH(CH₃)₂;
R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl.

19. A compound represented by formula VI or a pharmaceutically acceptable salt thereof 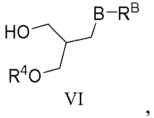 preferably a compound represented by formula VI-1 or a pharmaceutically acceptable salt thereof, 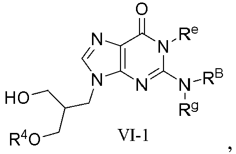 **characterized in that**
R⁴ is a hydroxyl protecting group, preferably trityl, tert-butyldimethylsilyl, tert-butyldiphenylsilyl, (trimethylsilyl)ethyl or 2-tetrahydropyran, and most preferably tert-butyldimethylsilyl or tert-butyldiphenylsilyl;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl, preferably -C(O)-CH₃, -C(O)-CH₂CH₃ or -C(O)-CH(CH₃)₂, and most preferably -C(O)-CH(CH₃)₂;
R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl.

20. A compound represented by formula VII or a pharmaceutically acceptable salt thereof 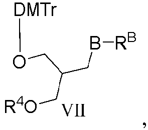 preferably a compound represented by formula VII-1 or a pharmaceutically acceptable salt thereof, 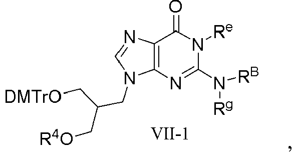 **characterized in that**
R⁴ is a hydroxyl protecting group, preferably trityl, tert-butyldimethylsilyl, tert-butyldiphenylsilyl, (trimethylsilyl)ethyl or 2-tetrahydropyran, and most preferably tert-butyldimethylsilyl or tert-butyldiphenylsilyl;
B is a base;
R^{B} is selected from -C(O)-C₁₋₆ alkyl, -C(O)-C₁₋₆ haloalkyl, -C(O)-C₁₋₆ hydroxy-substituted alkyl or -C(O)-C₁₋₆ amino-substituted alkyl, preferably -C(O)-CH₃, -C(O)-CH₂CH₃ or -C(O)-CH(CH₃)₂, and most preferably -C(O)-CH(CH₃)₂;
R^{e} and R^{g} are the same or different, and are each independently selected from hydrogen, halogen, hydroxyl, amino, C₁₋₆ haloalkyl, C₁₋₆ hydroxy-substituted alkyl or C₁₋₆ amino-substituted alkyl.

21. Use of the method according to any one of claims 1 to 14, the compound represented by formula II or the compound represented by formula II-1 according to claim 15, the compound represented by formula III or the compound represented by formula III-1 according to claim 16, the compound represented by formula IV or the compound represented by formula IV-1 according to claim 17, the compound represented by formula V or the compound represented by formula V-1 according to claim 18, the compound represented by formula VI or the compound represented by formula VI-1 according to claim 19, and the compound represented by formula VII or the compound represented by formula VII-1 according to claim 20 in the preparation of an oligonucleotide analogue, such as in the preparation of an siRNA.

22. A method for preparing a compound of formula 1, **characterized in that** the method comprises the steps of the method according to any one of claims 1 to 14,
